# EUROPEAN PATENT APPLICATION

(11) **EP 4 628 055 A1**
(43) Date of publication of application: **08.10.2025**
(21) Application number: 23900639.8
(22) Date of filing: 05.12.2023
(51) Int. Cl.: A61F 2/90, A61F 2/07, A61F 2/86, A61M 29/02

(54) **IMPLANT-TYPE MEDICAL DEVICE AND TRANSMISSION DEVICE**

(30) Priority: 06.12.2022 JP 2022194642; 06.12.2022 JP 2022194643
(71) Applicant: Terumo Kabushiki Kaisha, Tokyo 151-0072 (JP)
(72) Inventor: SAWADA, Satoshi, Ashigarakami-gun, Kanagawa 259-0151 (JP); YAMAGUCHI, Hiroshi, Ashigarakami-gun, Kanagawa 259-0151 (JP); SUEHARA, Satoru, Ashigarakami-gun, Kanagawa 259-0151 (JP)
(74) Representative: Prüfer & Partner mbB Patentanwälte · Rechtsanwälte
(86) International application number: PCT/JP2023/043390
(87) International publication number: WO 2024/122524

(57) **Abstract**

A blood vessel is efficiently extended in different directions to apply effective mechanical stimulation to a stimulation target site (for example, a baroreceptor of a blood vessel).

An implantable medical device 1 includes the blood vessel holding unit 10 capable of expanding and contracting to apply mechanical stimulation to the blood vessel wall of a blood vessel Bv, the blood vessel holding unit 10 has an annular structure in which a wavy linear component including at least one or more first curved portions 11 protruding toward the proximal end side, at least one or more second curved portions 11 protruding toward the distal end side, and at least one or more linear first connecting portions 13 connecting the first curved portion 11 and the second curved portion 12 is annular, the first curved portion 11 forms at least one or more first apex portions 11' in the radial direction of the central axis C of the annular structure, the second curved portion 12 forms at least one or more second apex portions 12' in the radial direction of the central axis C of the annular structure, and the first apex portion 11' and the second apex portion 12' are arranged to be shifted in the circumferential direction around the central axis C of the annular structure and in the long axis direction of the central axis C of the annular structure.

## Description

### Technical Field

The present invention relates to an implantable medical device and a delivery device for indwelling the medical device in a living body.

### Background Art

There is provided a mechanism (autonomic nervous system) for keeping the blood pressure constant, such as dilating blood vessels to lower the blood pressure when the blood pressure rises. However, for example, in a case where the function of the autonomic nerve that innervates the blood vessels or the blood vessels is deteriorated, the blood vessels are not expanded well even if the blood pressure rises, and the patient exhibits the symptom of hypertension.

Baroreceptor reflex is known as one of nervous system modulation of blood pressure. The baroreceptor reflex is a circulatory reflex that constantly monitors blood pressure by a baroreceptor and transmits the blood pressure to the central nervous system, and immediately corrects fluctuation of the blood pressure, and for example, mechanical stimulation is applied to the baroreceptor in the carotid sinus of the carotid artery from the inside of a blood vessel to act on the vagus nerve (such as the glossopharyngeal nerve) around the blood vessel, and the state of hypertension can be input to the living body in a pseudo manner.

Patent Literature 1 discloses a device including a plurality of adjacent longitudinal columns joined with a plurality of transverse members to define one or more openings extending between a plurality of adjacent longitudinal columns and a plurality of connecting members extending from the plurality of adjacent longitudinal columns to define a plurality of windows sized to increase pulsatility of a blood vessel wall for stimulating a baroreceptor in the blood vessel wall to lower blood pressure.

### Citation List

### Patent Literature

Patent Literature 1: US 10786372

### Summary of Invention

### Technical Problem

In the device of Patent Literature 1, the blood vessel is formed into a polygonal shape (such as a square or a pentagon), but the number of stimulation points on the blood vessel wall depends on the cross-sectional shape of the device, and is, for example, four for a quadrangle and five for a pentagon. In order to promote a decrease in blood pressure, it can be considered that it is useful to extend the blood vessel in different directions by increasing the number of stimulation points with respect to the blood vessel wall so that mechanical stimulation can be more effectively applied to the baroreceptor. In addition, when the blood vessel is not uniformly cylindrical, the expansion obtained in the radial direction is not uniform. Therefore, in the device of Patent Literature 1, there is a possibility that mechanical stimulation to the baroreceptor is small and a sufficient therapeutic effect cannot be obtained, and there is room for improvement.

At least one embodiment of the present invention has been made in view of the above circumstances, and specifically, it is an object of the present invention to provide an implantable medical device capable of efficiently extending a blood vessel in different directions to apply effective mechanical stimulation to a stimulation target site, and a delivery device for indwelling the medical device in a living body.

### Solution to Problem

The present invention is achieved by the following configuration.

(1) An implantable medical device indwelled in a blood vessel, in which the implantable medical device includes a blood vessel holding unit that is capable of expanding and contracting to apply mechanical stimulation to a blood vessel wall of the blood vessel, the blood vessel holding unit has an annular structure in which a wavy linear component including at least one or more first curved portions protruding toward a proximal end side, at least one or more second curved portions protruding toward a distal end side, and at least one or more linear first connecting portions connecting the first curved portion and the second curved portion is annular, and the first curved portion forms at least one or more first apex portions in a radial direction of a central axis of the annular structure, the second curved portion forms at least one or more second apex portions in the radial direction of the central axis of the annular structure, and the first apex portion and the second apex portion are arranged to be shifted in a circumferential direction around the central axis of the annular structure and in a long axis direction of the central axis of the annular structure.
(2) The implantable medical device according to (1), in which the blood vessel holding unit includes a distal end holding portion having the annular structure and positioned at a distal end of the blood vessel holding unit, a proximal end holding portion having the annular structure and positioned at a proximal end of the blood vessel holding unit, and at least one or more linear link portions connecting the first curved portion of the distal end holding portion and the second curved portion of the proximal end holding portion.
(3) In the implantable medical device according to (2), in which in the blood vessel holding unit, a number of the first curved portions and the second curved portions of the distal end holding portion in the circumferential direction and a number of the first curved portions and the second curved portions of the proximal end holding portion in the circumferential direction are same.
(4) In the implantable medical device according to (2), in which in the blood vessel holding unit, a number of the first curved portions and the second curved portions of the distal end holding portion in the circumferential direction and a number of the first curved portions and the second curved portions of the proximal end holding portion in the circumferential direction are different.
(5) The implantable medical device according to any of (2) to (4), in which the link portion includes a deformable portion that deforms so as to partially protrude toward the blood vessel wall at a time of expansion.
(6) The implantable medical device according to any one of claims (2) to (5), in which the link portion connects the first curved portion of the distal end holding portion and the second curved portion of the proximal end holding portion to be connected by a plurality of linear members extending in the axial direction.
(7) The implantable medical device according to any one of (2) to (6), in which the link portion extends in a twisted manner in a direction different from the central axis.
(8) The implantable medical device according to any one of (1) to (7), including at least one accessory unit having a V-shape including a base portion and both leg portions having a pair of leg portions extending from a proximal end of the base portion, respective proximal ends of the leg portions being individually connected to the first connecting portions adjacent in the circumferential direction.
(9) The implantable medical device according to any one of (1) to (8), in which the blood vessel holding unit includes a protruding portion partially deformed and protruding toward the blood vessel wall side.
(10) The implantable medical device according to any of (1) to (9), in which numbers of the first curved portions and the second curved portions formed are at least two or more.
(11) The implantable medical device according to any one of (1) to (9), in which numbers of both the first curved portions and the second curved portions formed are two or more and twenty or less.
(12) The implantable medical device according to any one of (1) to (9), in which numbers of both the first curved portions and the second curved portions formed are four or more and eight or less.
(13) The implantable medical device according to any of (1) to (12), in which the blood vessel is a carotid sinus of an internal carotid artery, and the blood vessel holding unit applies mechanical stimulation to a baroreceptor at the carotid sinus.
(14) The implantable medical device according to any of (1) to (13), including a collecting unit to be caught by a collecting device that collects the implantable device from inside a living body on a proximal end side of the blood vessel holding unit.
(15) A delivery device that delivers the implantable medical device according to any one of (1) to (14) to an indwelling position in a living body and indwells the implantable medical device in the living body, in which the implantable medical device includes a coupling unit in which a distal end portion is connected to a proximal end of the blood vessel holding unit adjacent in an axial direction, and a proximal end portion is aggregated so as to gradually reduce in diameter from the proximal end of the blood vessel holding unit toward a proximal end side and arranged at a position shifted from a center of the blood vessel, the delivery device includes a catheter having a lumen and a delivery member having an attachment that is relatively movable in the axial direction with respect to the catheter and to which the implantable medical device is attached, and the attachment portion includes a support portion having an inclined surface formed to match an outer shape of the coupling unit of the implantable medical device and supporting the coupling unit, a column portion extending from the inclined surface of the support portion toward a distal end side in the axial direction and inserted into the implantable medical device, and a pressing portion provided at a distal end of the support portion and in contact with a distal end side of the blood vessel holding unit of the implantable medical device.

### Advantageous Effects of Invention

With an implantable medical device of one embodiment of the present invention, it is possible to efficiently extend a blood vessel in different directions to apply effective mechanical stimulation to a stimulation target site (for example, a baroreceptor of a blood vessel).

Further, a delivery device of one embodiment of the present invention has an inclined surface that supports a coupling unit to match an outer shape of the implantable medical device, and thus the implantable medical device can be disposed between a support portion and a pressing portion in a state of being inserted into a column portion, and can be delivered without falling off to an indwelling position in a state of holding both end sides of the implantable medical device.

### Brief Description of Drawings

Fig. 1 is a developed view of an implantable medical device according to a first embodiment.
Fig. 2 is a side view of the implantable medical device according to the first embodiment.
Fig. 3A is a schematic perspective view illustrating a positional relationship between a first curved portion and a second curved portion of a blood vessel holding unit.
Fig. 3B is a view illustrating a positional relationship between the first curved portion and the second curved portion when the blood vessel holding unit is viewed from a distal end side (front side) in an axial direction.
Fig. 4A is a view illustrating a first cell of the implantable medical device according to the first embodiment.
Fig. 4B is a view illustrating a second cell of the implantable medical device according to the first embodiment.
Fig. 5 is a configuration view of a delivery device.
Fig. 6 is a configuration view illustrating a state at the time of indwelling the implantable medical device in the delivery device illustrated in Fig. 5.
Fig. 7A is a view illustrating a form of a sliding hole formed in an operation unit of the delivery device.
Fig. 7B is a view illustrating another form of the sliding hole formed in the operation unit of the delivery device.
Fig. 7C is a view illustrating another form of the sliding hole formed in the operation unit of the delivery device.
Fig. 8A is a configuration view illustrating a form of a collecting device.
Fig. 8B is a configuration view illustrating another embodiment of the collecting device.
Fig. 8C is a configuration view illustrating another embodiment of the collecting device.
Fig. 9A is a view illustrating an operation at the time of indwelling the implantable medical device according to the first embodiment.
Fig. 9B is a view illustrating an operation at the time of indwelling the implantable medical device according to the first embodiment.
Fig. 9C is a view illustrating an operation at the time of indwelling the implantable medical device according to the first embodiment.
Fig. 9D is a view illustrating an operation at the time of indwelling the implantable medical device according to the first embodiment.
Fig. 9E is a view illustrating an operation at the time of indwelling the implantable medical device according to the first embodiment.
Fig. 9F is a view illustrating an operation at the time of indwelling the implantable medical device according to the first embodiment.
Fig. 10A is a developed view illustrating a modification of the implantable medical device according to the first embodiment.
Fig. 10B is a developed view illustrating another modification of the implantable medical device according to the first embodiment.
Fig. 11 is a developed view illustrating another modification of the implantable medical device according to the first embodiment.
Fig. 12 is a developed view of an implantable medical device according to a second embodiment.
Fig. 13A is a view illustrating a first cell of an implantable medical device according to the second embodiment.
Fig. 13B is a view illustrating a second cell of the implantable medical device according to the second embodiment.
Fig. 14A is a developed view of an implantable medical device according to a third embodiment.
Fig. 14B is a developed view illustrating a modification of the implantable medical device according to the third embodiment.
Fig. 15 is a developed view of an implantable medical device according to a fourth embodiment.
Fig. 16 is a developed view illustrating a modification of the implantable medical device according to the fourth embodiment.
Fig. 17 is a developed view illustrating another modification of the implantable medical device according to the fourth embodiment.
Fig. 18 is a developed view illustrating another modification of the implantable medical device according to the fourth embodiment.
Fig. 19 is a developed view of an implantable medical device according to a fifth embodiment.

### Description of Embodiments

Hereinafter, a mode for carrying out the present invention will be described in detail with reference to the drawings. Embodiments herein are illustrated to embody the technical idea of the present invention and do not limit the present invention. Other embodiments, examples, technical operations, and the like that could be conceived by those skilled in the art without departing from the gist of the invention are all included in the scope and gist of the invention and included in the invention disclosed in the claims and the scope of equivalents thereof.

Furthermore, for the purpose of illustration and for ease of comprehension, the scale, aspect ratio, shape, and the like in the drawings attached may be changed from actual ones as appropriate and illustrated schematically. However, it is noteworthy that the drawings are examples and do not limit the interpretation of the present invention.

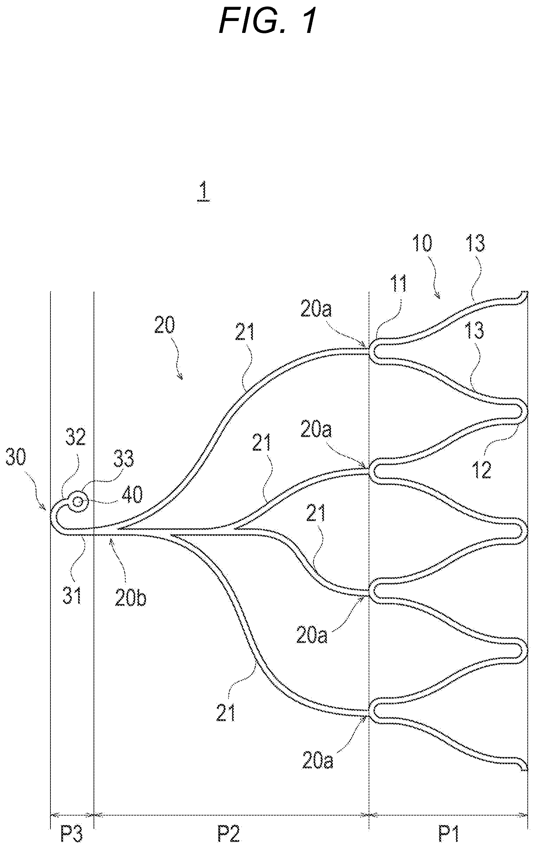

In the present specification, the following directions are defined for convenience of description. In Fig. 1, the "long axis direction" is a longitudinal direction (left-right direction in the drawing) of an implantable medical device 1 and is a direction along a central axis C of the implantable medical device 1. The "radial direction" is a direction away from or approaching the central axis C of the implantable medical device 1. The "circumferential direction" is a rotation direction with the central axis C of the implantable medical device 1 as a reference axis. Note that the central axis C of the implantable medical device 1 coincides with a central axis of the blood vessel holding unit 10.

In Fig. 1, a "distal end" is a side (right side in the drawing) on which the blood vessel holding unit 10 is provided and which is inserted into a blood vessel Bv, and a side opposite to the distal end (side on which a collecting unit 30 on the left side in the figure is disposed) is a "proximal end".

In the following description, note that ordinal numerals such as "first" and "second" will be given but are used for convenience's sake and do not define any order unless otherwise specified.

The implantable medical device 1 according to the present embodiment is a medical instrument that is indwelled in a biological lumen such as a blood vessel Bv, applies mechanical stimulation to a stimulation target site (for example, a baroreceptor) to act on the vagus nerve, and expands, presses, and stimulates the stimulation target site from the inside of the blood vessel wall in order to treat a predetermined disease. Note that the implantable medical device 1 can apply mechanical stimulation to a stimulation target site in a biological lumen other than the blood vessel Bv.

In the following embodiment, the implantable medical device 1 is indwelled in a carotid sinus near an entrance of an internal carotid artery branching from a common carotid artery, and will be described as an instrument used for a treatment (so-called hypotensive treatment using baroreceptor reflex) of applying mechanical stimulation to a baroreceptor in the carotid sinus to send an impulse to a cardiovascular center, and effectively decreasing heart rate and blood pressure from the cardiovascular center via parasympathetic stimulation.

### [First Embodiment]

A configuration of an implantable medical device 1 according to a first embodiment will be described with reference to Figs. 1 to 4.

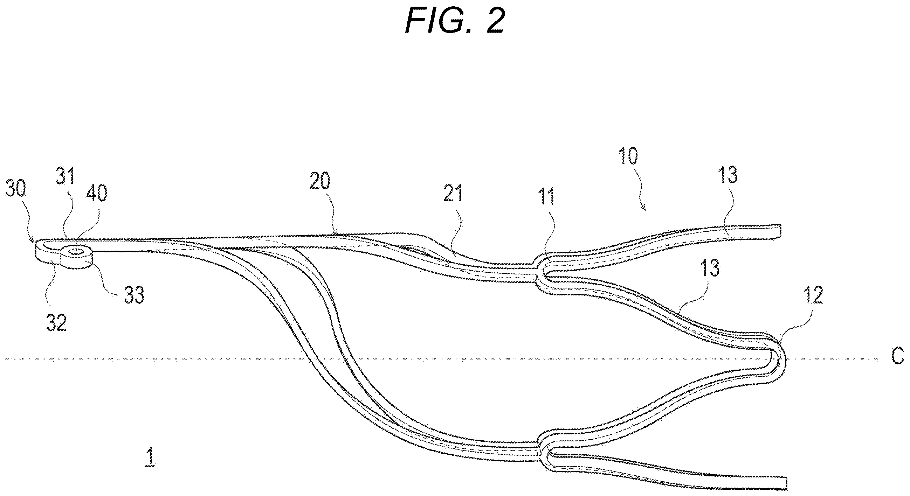

As illustrated in Fig. 1 or 2, the implantable medical device 1 according to the first embodiment includes a blood vessel holding unit 10 disposed on the distal end side, a coupling unit 20 connected to a proximal end of the blood vessel holding unit 10, and a collecting unit 30 connected to a proximal end of the coupling unit 20. As illustrated in Fig. 1, the blood vessel holding unit 10 of the implantable medical device 1 is a P1 portion, the coupling unit 20 is a P2 portion, and the collecting unit 30 is a P3 portion. The implantable medical device 1 is a hollow member formed to be expandable and contractible by a linear component, and expands when being indwelled in the blood vessel Bv to press the blood vessel Bv from the inside of the blood vessel wall to apply mechanical stimulation.

The implantable medical device 1 can be indwelled at a predetermined location in the blood vessel Bv using a delivery device 100 described later. Further, after being indwelled in the blood vessel Bv, the implantable medical device 1 can be removed ex vivo using a collecting device 200 described later.

### <Blood vessel holding unit>

As illustrated in Figs. 1 and 2, the blood vessel holding unit 10 has an annular structure including linear components. The blood vessel holding unit 10 has an annular structure at least on the proximal end side.

The annular structure includes at least one or more first curved portions 11 protruding toward the proximal end side, at least one or more second curved portions 12 protruding toward the distal end side, and at least one or more linear first connecting portions 13 connecting the first curved portions 11 and the second curved portions 12.

One end of the first curved portion 11 is connected to an end of one first connecting portion 13 adjacent in the circumferential direction, and the other end of the first curved portion 11 is connected to an end of the other first connecting portion 13 adjacent in the circumferential direction. Further, one end of the second curved portion 12 is connected to an end of one first connecting portion 13 adjacent in the circumferential direction, and the other end of the second curved portion 12 is connected to an end of the other first connecting portion 13 adjacent in the circumferential direction. The annular structure has a wave shape in which the first curved portion 11, the first connecting portion 13, and the second curved portion 12 are alternately arranged in this order in the circumferential direction.

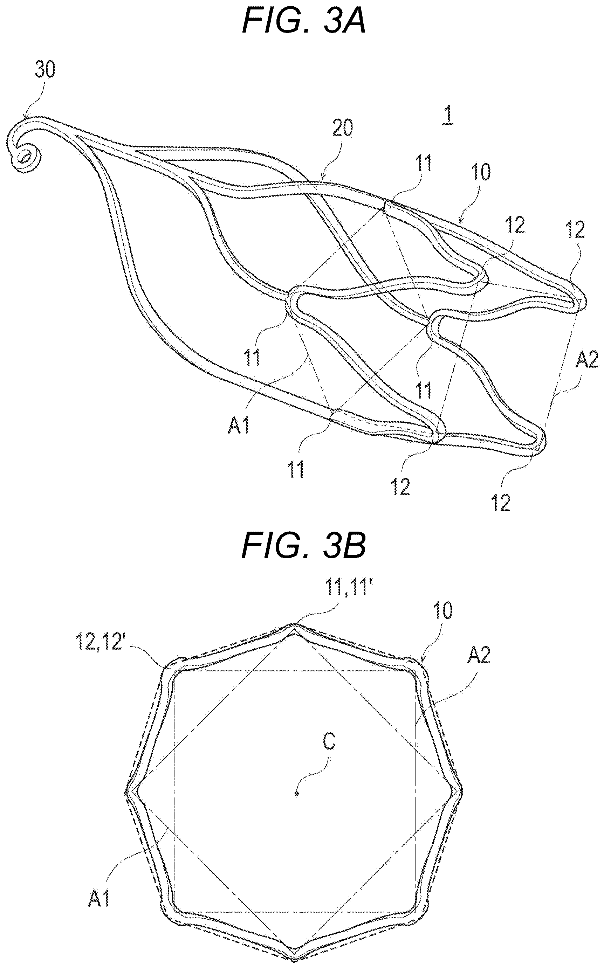

As illustrated in Figs. 3A and 3B, the blood vessel holding unit 10 is arranged so that phases in the circumferential direction around the central axis C of the first curved portions 11 and the second curved portions 12 arranged at both ends of the first connecting portion 13 are shifted from each other. That is, the first curved portions 11 form at least two or more first apex portions 11' in the radial direction of the central axis C, the second curved portions 12 form at least two or more second apex portions 12' in the radial direction of the central axis C, and the first apex portions 11' and the second apex portions 12' are arranged to be shifted in the circumferential direction around the central axis C and the long axis direction of the central axis C. As illustrated in Fig. 3B, in the blood vessel holding unit 10, when viewed from the distal end side in the axial direction (front side), a figure A1 defined by connecting the first curved portions 11 and a figure A2 defined by connecting the second curved portions 12 are both quadrangles (one-dot chain lines in the drawing), and when apex positions of the respective quadrangles, that is, the first apex portions 11' and the second apex portions 12' are connected, an octagon (a dotted line in the drawing) is formed.

In the blood vessel holding unit 10, the positions of the first curved portions 11 and the second curved portions 12 are shifted in the circumferential direction, so that the first apex portions 11' and the second apex portions 12' are arranged to protrude, and the number of contact points with the blood vessel wall can be increased. Therefore, when the blood vessel holding unit 10 is indwelled in the blood vessel Bv, the number of stimulation points on the blood vessel Bv is increased as compared with a stent or the like having a general cylindrical shape and a non-shifted phase, and the blood vessel Bv extends in different directions, so that the probability that mechanical stimulation can be applied to a baroreceptor that is a stimulation target site in the blood vessel Bv increases, and the mechanical stimulation can be efficiently applied.

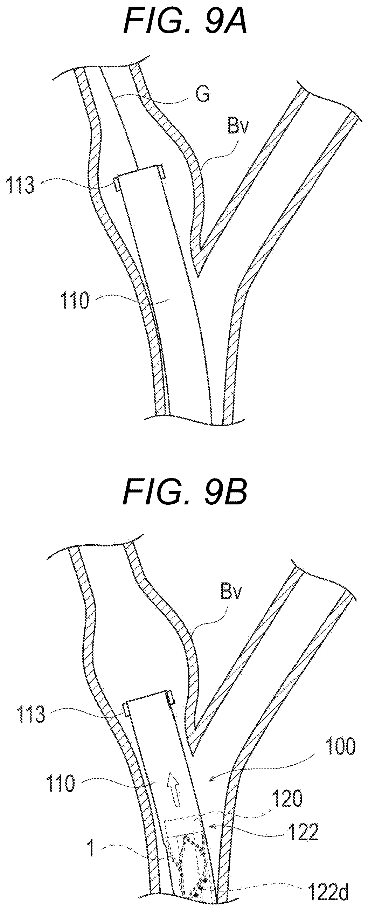

As illustrated in Fig. 1, the first curved portions 11 formed on the proximal end side of the blood vessel holding unit 10 are individually connected to the coupling unit 20. Therefore, when the blood vessel holding unit 10 is indwelled in the blood vessel Bv, there is no site protruding on the proximal end side (P2 portion and P3 portion) of the implantable medical device 1. That is, the proximal end side of the implantable medical device 1 is configured so that the implantable medical device 1 does not exist in a part of the circumferential direction of the central axis C, and the distal end side of the implantable medical device 1 is configured so that the implantable medical device 1 exists in the entire circumferential direction of the central axis C, and the implantable medical device 1 is a three-dimensional body in the shape of an obliquely truncated cylinder (a right cylinder with one end obliquely cut off). Therefore, when the implantable medical device 1 is indwelled in the blood vessel Bv, it does not cover other blood vessels Bv in the vicinity. When the implantable medical device 1 is indwelled in the carotid sinus of the internal carotid artery as illustrated in Figs. 9A to 9F, a part of the blood vessel holding unit 10 can be prevented from covering the external carotid artery branching from the common carotid artery.

The numbers of both the first curved portions 11 and the second curved portions 12 formed can be at least one or more, preferably two or more, more preferably two or more and twenty or less, and still more preferably four or more and eight or less. With such a configuration, it is possible to appropriately hold the blood vessel Bv from the inside and effectively apply the mechanical stimulation.

### <Coupling unit>

As illustrated in Figs. 1 and 2, a distal end of the coupling unit 20 is connected to the proximal end (first curved portion 11) of the blood vessel holding unit 10, and a proximal end is connected to a proximal end of the collecting unit 30. The coupling unit 20 can be configured by a plurality of linear members 21 extending in the axial direction. As illustrated in Fig. 1, since there are four first curved portions 11 at the proximal end of the blood vessel holding unit 10 to be connected, the coupling unit 20 includes four linear members.

As illustrated in Fig. 2, in the coupling unit 20, a distal end portion 20a is connected to the first curved portion 11 at the proximal end of the blood vessel holding unit 10 adjacent in the axial direction, and proximal end portions 20b are aggregated so as to gradually reduce in diameter from the proximal end of the blood vessel holding unit 10 toward the proximal end side and so as to be away from the central axis C in the radial direction, and arranged at a position shifted from the center of the blood vessel Bv. That is, the respective proximal end portions 20b of the coupling unit 20 are aggregated at positions shifted from the center of the blood vessel Bv.

The proximal end portions of the coupling unit 20 are arranged to be shifted from at least the center of the blood vessel Bv. This is to reduce adverse effects (inhibition of blood flow, risk of generating thrombus, and the like) of arranging the blood vessel Bv at the center of the blood vessel Bv, and the effect is larger as the distance from the center of the blood vessel Bv in the radial direction increases, and the blood vessel Bv is preferably arranged near the blood vessel wall.

### <Collecting unit>

The collecting unit 30 extends from the proximal end portion 20b of the coupling unit 20 to the proximal end side along the axial direction, and is caught by the collecting device 200 that collects the implantable medical device 1 from inside the living body. The collecting unit 30 has a shape and a function that can be collected by the collecting device 200.

The collecting unit 30 can be configured to include an extending portion 31 connected to the proximal end portion 20b of the coupling unit 20 and extending in the axial direction, and an engaging portion 32 bent in a U shape from the proximal end toward the distal end side of the extending portion 31. The engaging portion 32 has a hook shape so as to be engageable with a catching portion 220 of the collecting device 200. The shape of the engaging portion 32 is not limited to the U shape, and may be any shape that can be easily engaged in accordance with the form of the catching portion 220 of the collecting device 200.

In the collecting unit 30, preferably, the direction of the engaging portion 32 is bent in a direction away from the blood vessel wall of the blood vessel Bv in consideration of ease of collection by the collecting device 200. Thus, a distal end of the engaging portion 32 can be separated from the blood vessel wall, so that the collecting device 200 can easily catch the blood vessel.

In the collecting unit 30, the engaging portion 32 can also be disposed along the blood vessel wall surface of the blood vessel Bv. Thus, since the engaging portion 32 uniformly touches the blood vessel wall surface, the collecting unit 30 can have a configuration in which there is no portion protruding in the radial direction of the blood vessel Bv that can obstruct the blood flow.

Further, the collecting unit 30 can also be configured to be magnetically attractable to the collecting device 200. In the collecting unit 30, a magnetic portion 33 that is magnetically attractable to the collecting device 200 can be provided in the extending portion 31 and/or the engaging portion 32. The magnetic portion 33 can be constituted by a magnet having a magnetic pole different from that of a magnetic member provided in the collecting device 200 or a magnetic attracting body such as a magnetic body (ferromagnetic body or the like) that is magnetically attractable when the magnetic member is a magnet.

The collecting unit 30 can be provided with a marker portion 40 having an X-ray contrast property. Thus, the implantable medical device 1 can adjust positioning at the time of indwelling and an arrangement position with respect to the delivery device 100 while confirming from outside the living body using a modality (various image diagnostic apparatuses such as an angiographic apparatus) or the like. Note that the marker portion 40 can be provided not only in the collecting unit 30 but also in the blood vessel holding unit 10 and the coupling unit 20. In a case where the marker portion 40 is provided in the blood vessel holding unit 10, it is preferable to provide the marker portion in the second curved portion 12 located on the opposite side of the collecting unit 30 located on the proximal end side of the implantable medical device 1 in order to easily grasp the entire shape of the implantable medical device 1. The second curved portion 12 only needs to be a curved portion constituting the blood vessel holding unit 10, and may be provided at one position or a plurality of positions where the marker portions 40 is provided.

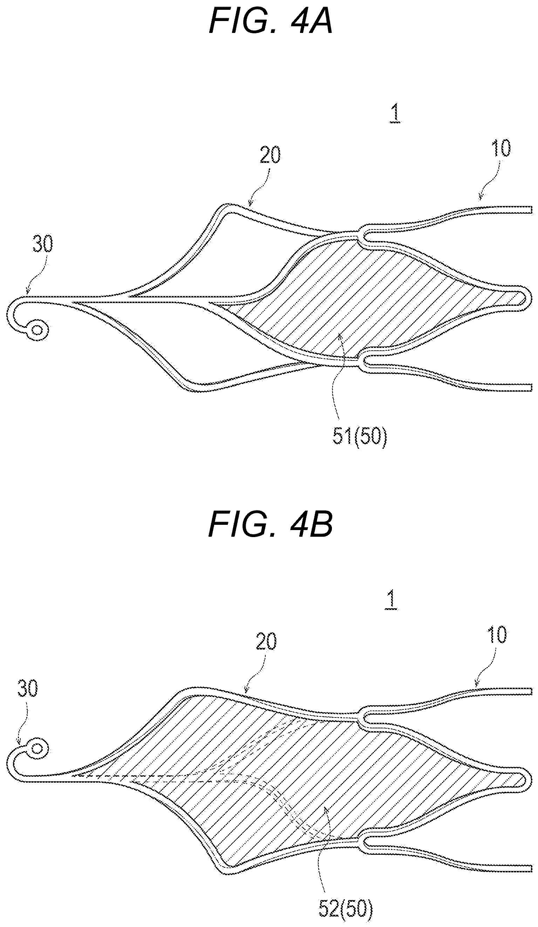

Figs. 4A and 4B illustrate cells 50 (a first cell 51 and a second cell 52) which are opening portions formed on a peripheral surface of the implantable medical device 1. As illustrated in Fig. 4A, in the implantable medical device 1, a first cell 51 surrounded by the first connecting portions 13 of the blood vessel holding unit 10 and the coupling unit 20 is formed on the peripheral surface. As illustrated in Fig. 4B, in the implantable medical device 1, a second cell 52 surrounded by the first connecting portions 13 of the blood vessel holding unit 10 and the coupling unit 20 and having a cell area different from that of the first cell 51 is formed on the peripheral surface. When the first cell 51 illustrated in Fig. 4A is compared with the second cell 52 illustrated in Fig. 4B, the second cell 52 has a larger cell area than the first cell 51. As described above, since the implantable medical device 1 has the cells 50 having different cell areas on the peripheral surface, even in a case where the implantable medical device 1 reaches a bifurcation, favorable blood flow passability can be obtained as compared with a form in which the cell area is uniform, and the risk that the strut of the implantable medical device 1 covers the bifurcation can be reduced in the first place.

The implantable medical device 1 is formed into a desired size and shape by laser processing each dimension (such as an outer diameter, an inner diameter, or an axial length) of a cylindrical base material. The implantable medical device 1 has a restoring force (self-expanding force) from a compressed state (contracted state) to an original shape (expanded state). Therefore, the base material is preferably a superelastic alloy having shape memory capability and biocompatibility, such as a titanium-nickel alloy. However, a polymer material or another metal material can be suitably used as necessary. Examples of the polymer material include polyolefins such as polyethylene and polypropylene, polyesters such as polyethylene terephthalate, and fluorine-containing polymers such as polytetrafluoroethylene and a tetrafluoroethylene-ethylene copolymer. Examples of the metal material include a cobalt-chromium alloy, stainless steel, iron, titanium, aluminum, tin, and a zinc-tungsten alloy. In addition, the surface of the implantable medical device 1 may be subjected to a surface treatment for imparting desired performance. Examples of the surface treatments can include an antithrombotic coating composed of a resin material such as parylene or polytetrafluoroethylene, a polymer material, or a biomaterial, and an anti-thrombogenic coating.

Since the implantable medical device 1 is a laser cut type manufactured by processing a base material with a laser, the implantable medical device 1 has a higher degree of freedom in design than a braided type in which one or a plurality of wires are interwoven, and is easily molded into a desired outer shape. Therefore, the collecting unit 30 can be easily processed into a shape that can be disposed near the blood vessel wall. Further, the laser cut type has a higher radial force and is easier to adjust than the braided type.

The implantable medical device 1 can be indwelled in the blood vessel Bv using the following delivery device 100.

### (Delivery device)

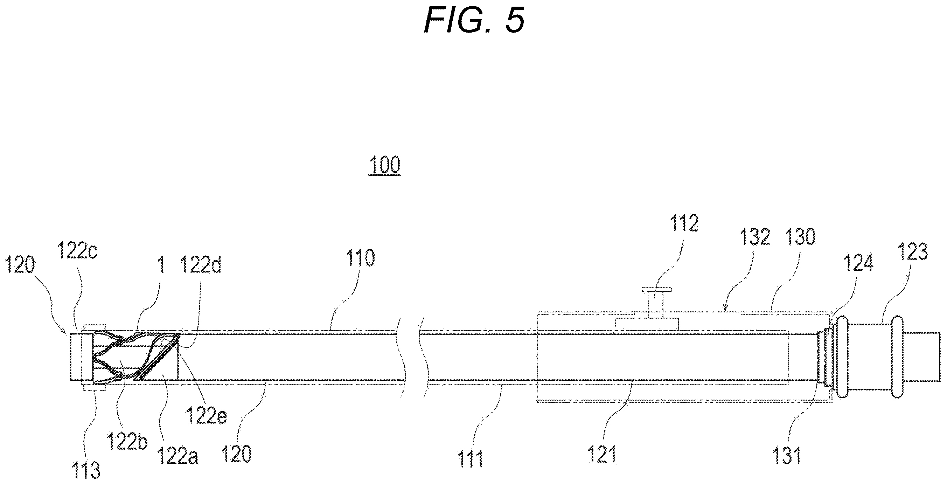

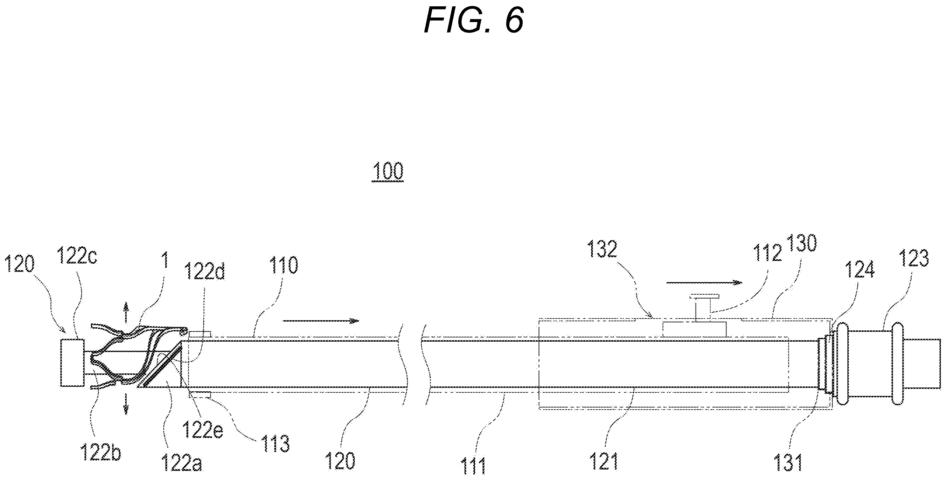

As illustrated in Figs. 5 and 6, the delivery device 100 includes a catheter 110, a delivery member 120, and an operation unit 130. The delivery device 100 can be introduced into the blood vessel Bv using an introduction device such as an introducer that is percutaneously inserted into a living body.

The catheter 110 includes a catheter body 111 formed of a tubular elongated member having a lumen. A lever portion 112 that is operated when the catheter body 111 is relatively moved with respect to the delivery member 120 is provided on an outer peripheral surface on the proximal end side of the catheter body 111. Further, a marker portion 113 having an X-ray contrast property is provided at the distal end of the catheter 110.

The lever portion 112 is provided so as to extend from an outer surface of the catheter body 111 in a direction intersecting the axial direction and away from the outer peripheral surface, and a part of the distal end protrudes from a sliding hole 132 of the operation unit 130. When the operator pinches the portion of the lever portion 112 protruding from the sliding hole 132 and slides along the sliding hole 132, the catheter 110 can be moved relative to the delivery member 120.

The delivery member 120 is inserted through an insertion port 114 at a proximal end of the catheter 110 and delivers the implantable medical device 1 to an indwelling position. The delivery member 120 includes an attachment portion 122 provided at a distal end of an elongated main body 121 to attach the implantable medical device 1, and a grip portion 123 provided at a proximal end of the main body 121 to be gripped when the delivery member 120 is inserted into the catheter 110.

The attachment portion 122 includes a support portion 122a having an inclined surface 122e formed to match an outer shape of the coupling unit 20 so that the coupling unit 20 of the implantable medical device 1 can be supported, a column portion 122b having a diameter smaller than that of the main body 121 and extending from the inclined surface 122e of the support portion 122a to the distal end side in the axial direction, a pressing portion 122c provided at a distal end of the column portion 122b and in contact with the distal end side of the blood vessel holding unit 10 of the implantable medical device 1, and a marker portion 122d provided in the support portion 122a.

The implantable medical device 1 is attached to the attachment portion 122 in a state in which an outer surface of the coupling unit 20 and the inclined surface 122e of the support portion 122a are brought into contact with each other, the column portion 122b is inserted into the implantable medical device 1, and the distal end of the blood vessel holding unit 10 and the pressing portion 122c are brought into contact with each other. Thus, since the implantable medical device 1 is disposed between the support portion 122a and the pressing portion 122c in a state of being inserted into the column portion 122b, both end sides are held, and falling off during delivery can be prevented. Further, since the implantable medical device 1 is attached to the attachment portion 122 in a state of being inserted into the column portion 122b, displacement in the radial direction at the time of delivery is suppressed, and the implantable medical device 1 can be smoothly delivered without being caught in a lumen of the catheter 110 during delivery.

A first fitting portion 124 that is inserted into and fitted to a second fitting portion 131 of the operation unit 130 described later is provided at a distal end of the grip portion 123. The first fitting portion 124 is fitted to the second fitting portion 131 after the delivery member 120 is inserted into the catheter 110 and the indwelling posture and the indwelling position of the implantable medical device 1 attached to the attachment portion 122 are adjusted. Thus, the implantable medical device 1 can be indwelled in the blood vessel Bv in a fixed indwelling posture. Note that, in Figs. 5 and 6, the shapes of both illustrating a form in which the first fitting portion 124 having a protruding shape is inserted into and fitted in the second fitting portion 131 having a recessed shape can be reversed. In this case, the first fitting portion 124 has a recessed shape, and the second fitting portion 131 has a protruding shape.

The operation unit 130 is disposed outside the living body and is operated by the operator. The second fitting portion 131 to be fitted in the first fitting portion 124 of the delivery member 120 is provided at a proximal end of the operation unit 130. The sliding hole 132 that guides a sliding direction of the lever portion 112 when a retracting operation of the catheter 110 is performed is formed in an upper surface of the operation unit 130.

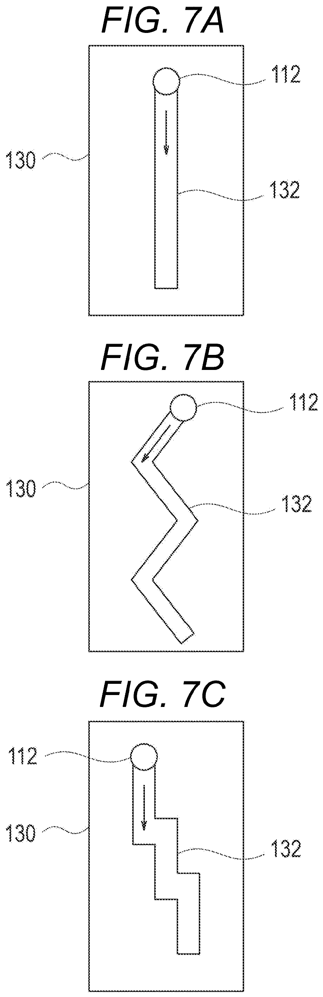

Figs. 7A to 7C illustrate the forms of the sliding hole 132. The sliding hole 132 can have a form formed linearly along the axial direction as illustrated in Fig. 7A, a form formed in a zigzag shape laterally along the axial direction as illustrated in Fig. 7B, and a form formed in a staircase shape as illustrated in Fig. 7C. Among these forms, the form illustrated in Figs. 7B and 7C is effective because the moving distance of the catheter 110 can be reduced with respect to the moving distance of the lever portion 112, and the moving distance of the catheter can be finely adjusted. Note that the forms of the sliding hole 132 are not limited to the forms illustrated in Figs. 7A to 7C.

The delivery device 100 inserts the delivery member 120 with the implantable medical device 1 attached thereto into the catheter 110 previously delivered to the vicinity of the indwelling position, adjusts the indwelling position of the implantable medical device 1 from the positions of the pressing portion 122c and the marker portion 122d, and brings about the state illustrated in Fig. 5. At this time, the first fitting portion 124 of the grip portion 123 is fitted to the second fitting portion 131. Thereafter, when a retracting operation is performed on the lever portion 112, the catheter 110 moves relative to the delivery member 120 to the proximal end side, and the attachment portion 122 is exposed in the blood vessel Bv. Then, the implantable medical device 1 is restored from the contracted state to the original shape by self-expansion. The implantable medical device 1 is indwelled while pressing the blood vessel wall of the blood vessel Bv at the indwelling position to provide mechanical stimulation. Note that the operation at the time of indwelling the implantable medical device 1 will be described in detail later.

The implantable medical device 1 can be recovered from the living body (in the blood vessel Bv) using the following collecting device 200.

### <Collecting device>

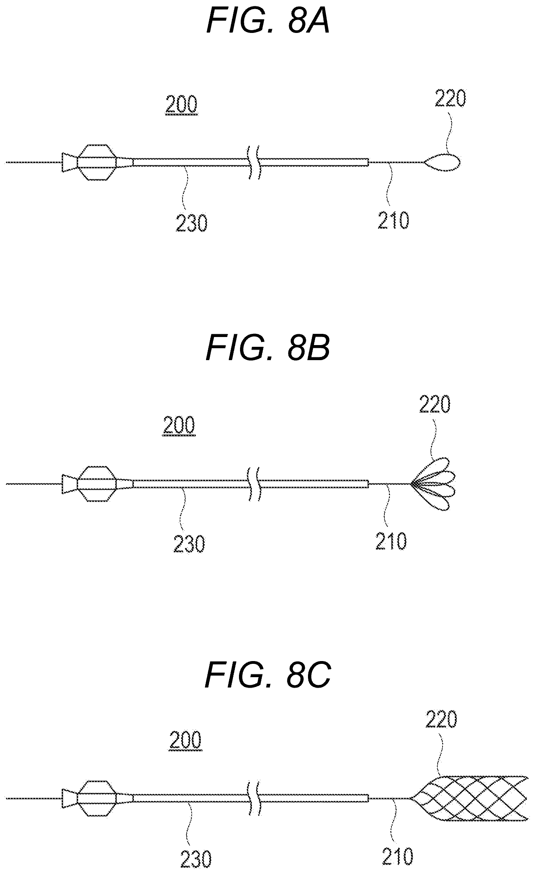

Figs. 8A to 8C illustrate a form of the collecting device 200 for collecting the implantable medical device 1 from the living body.

The collecting device 200 is a device for collecting the implantable medical device 1 indwelled in the blood vessel Bv. The collecting device 200 has a configuration and a function capable of collecting the implantable medical device 1 from inside the living body by engaging with the engaging portion 32 of the collecting unit 30 provided on the proximal end side of the implantable medical device 1.

As illustrated in Fig. 8A, the collecting device 200 can be configured to include an elongated main body portion 210 and an annular catching portion 220 provided at a distal end of the main body portion 210. As illustrated in Fig. 8B, the collecting device 200 can be configured to include the long main body portion 210 and the catching portion 220 provided at the distal end of the main body portion 210 and including a plurality of annular portions expanding at predetermined intervals in the radial direction. As illustrated in Fig. 8C, the collecting device 200 may include the elongated main body portion 210 and a net-like catching portion 220 provided at a distal end of the main body 210 and expanding in the radial direction. Further, the collecting device 200 includes an elongated collecting catheter 230 having a lumen for drawing the implantable medical device 1 to be collected.

In any of the forms illustrated in Figs. 8A to 8C, the collecting device 200 can collect the implantable medical device 1 by engaging the catching portion 220 with the engaging portion 32 of the implantable medical device 1. Further, the collecting device 200 can catch the collecting unit 30 by magnetic attraction in addition to the engagement with the engaging portion 32 by providing the engaging portion 32 with a magnetic attraction property and providing the catching portion 220 with a magnetic attraction property. In a case where the catching portion 220 is provided with a magnetic attraction property, the catching portion 220 itself may be formed of a magnetic attracting body, or the magnetic attracting body may be formed on the outer surface or the inside of the catching portion 220. Note that the form of the collecting device 200 is not limited to the forms illustrated in Figs. 8A to 8C as long as the implantable medical device 1 has a collectable configuration and function.

### [Operation]

Next, an operation at the time of indwelling the implantable medical device 1 according to the first embodiment will be described with reference to Figs. 9A to 9F as appropriate. The operation illustrated in Figs. 9A to 9F illustrates an operation when the implantable medical device 1 is indwelled in the carotid sinus of the internal carotid artery branching from the common carotid artery. Note that the place where the implantable medical device 1 is indwelled is not limited to the internal carotid artery, and the implantable medical device 1 can be appropriately indwelled in a biological lumen of the patient according to the therapeutic purpose.

As illustrated in Fig. 9A, the operator inserts the catheter 110 of the delivery device 100 into the blood vessel Bv along a guide wire G via an introduction device such as an introducer. Since the marker portion 113 is provided at the distal end of the catheter 110, the operator can insert the catheter 110 while confirming a medical image obtained by imaging the position of the marker portion 113 with the modality. At this time point, the guide wire G may be removed or may remain indwelled as illustrated in the drawing.

Next, as illustrated in Fig. 9B, the operator attaches the implantable medical device 1 to the attachment portion 122 of the delivery member 120, inserts the implantable medical device 1 from the proximal end side of the catheter 110, and delivers the implantable medical device 1 to the vicinity of the indwelling position.

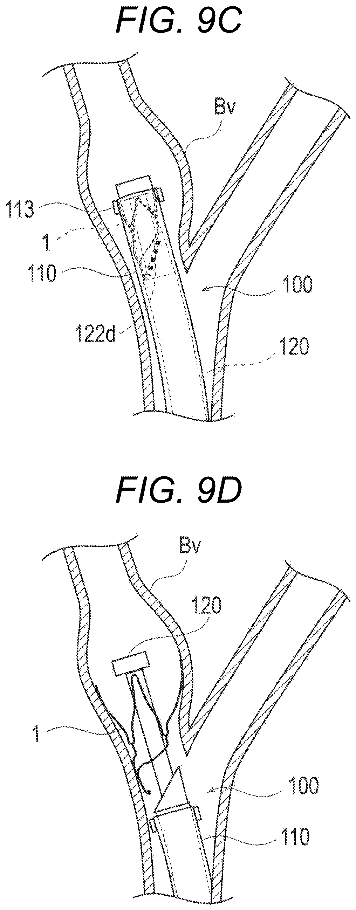

Next, as illustrated in Fig. 9C, the operator adjusts the indwelling position of the implantable medical device 1 from the position of the marker portion 122d of the delivery member 120. When the adjustment of the indwelling position of the implantable medical device 1 is completed, the operator fits the first fitting portion 124 of the grip portion 123 to the second fitting portion 131 to fix the position of the delivery member 120.

Next, as illustrated in Fig. 9D, the operator pulls the lever portion 112 of the catheter 110 to the proximal end side to perform the retracting operation of the catheter 110, and retracts the distal end of the catheter 110 to the proximal end side. The catheter 110 is relatively moved to the proximal end side with respect to the delivery member 120 by the retracting operation, and the attachment portion 122 is exposed in the blood vessel Bv. Thus, the implantable medical device 1 self-expands and restores from the contracted state to the original shape (expanded state).

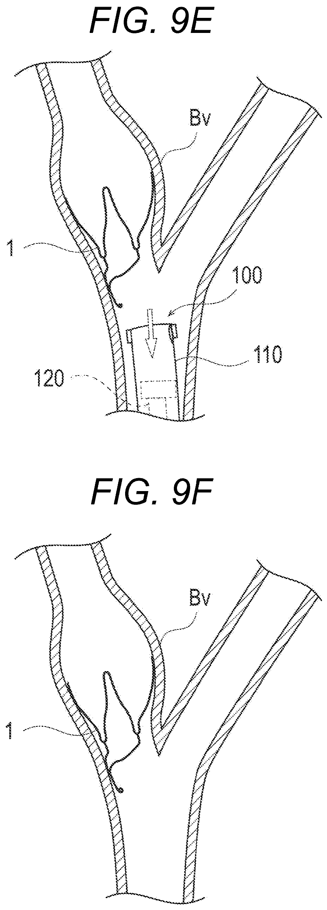

Then, when confirming that the implantable medical device 1 is expanded, the surgeon removes the catheter 110 and the delivery member 120 from inside the living body as illustrated in Fig. 9E. Thus, the implantable medical device 1 is indwelled in the carotid sinus as a target indwelling position as illustrated in Fig. 9F. The implantable medical device 1 indwelled in the blood vessel Bv is indwelled while maintaining a state in which the blood vessel wall is pressed from the inside to press the blood vessel wall from the inside to apply mechanical stimulation to the baroreceptor of the carotid sinus as the stimulation target site. Thus, the baroreceptor mechanically stimulated by the implantable medical device 1 acts on the cardiac center via the glossopharyngeal nerve, and can suppress the heart rate via the autonomic nerve to lower the blood pressure. That is, it is possible to implement hypotensive treatment using the vagus nerve reflex. In addition, the present invention can be applied to treatment via control of an inflammatory response caused by stimulation of the vagus nerve. In the embodiment described above, the catheter 110 is arranged in advance in the blood vessel Bv, and the delivery member 120 to which the implantable medical device 1 is attached is introduced from the proximal end thereof, but an assembly in which the implantable medical device 1 or the delivery member 120 is loaded in the catheter 110 may be introduced into the blood vessel Bv.

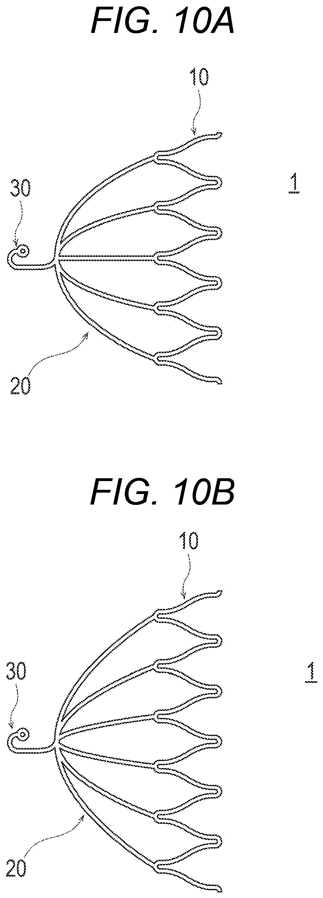

In the implantable medical device 1 of the first embodiment, as illustrated in Figs. 10A and 10B, the number of the first curved portions 11, the second curved portions 12, and the first connecting portions 13 connected to the first curved portions 11 and the second curved portions 12 can be arbitrarily set.

As illustrated in Fig. 10A, the implantable medical device 1 can have a configuration in which five first curved portions 11 and five second curved portions 12 are arranged as a modification. In the implantable medical device 1 illustrated in Fig. 10A, figures defined by connecting the first curved portions 11 and the second curved portions 12 are both a pentagon. The pentagon having the first curved portions 11 as apexes and the pentagon having the second curved portions 12 as apexes are shifted in phase in the circumferential direction, and thus form a decagon when apex positions of the respective pentagons are connected as viewed from the axial direction.

As illustrated in Fig. 10B, the implantable medical device 1 can have a configuration in which six first curved portions 11 and six second curved portions 12 are arranged as a modification. In the implantable medical device 1 illustrated in Fig. 10B, figures defined by connecting the first curved portions 11 and the second curved portions 12 are both a hexagon. The hexagon having the first curved portions 11 as apexes and the hexagon having the second curved portions 12 as apexes are shifted in phase in the circumferential direction, and thus form a dodecagon when apex positions of the respective hexagons are connected as viewed from the axial direction.

In the forms illustrated in Figs. 10A and 10B, since the number of contact points with the blood vessel wall is increased as compared with the embodiment illustrated in Fig. 1, the mechanical stimulation can be more effectively applied to the blood vessel Bv.

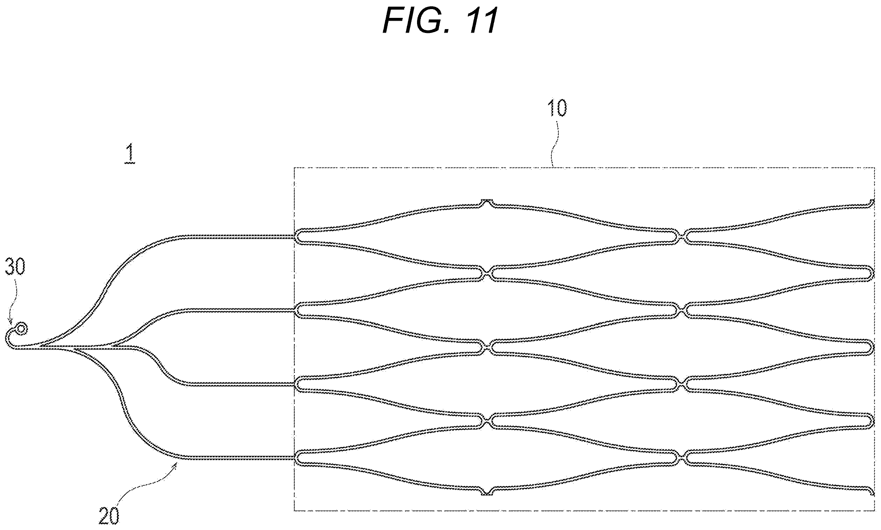

As illustrated in Fig. 11, in the implantable medical device 1 of the first embodiment, as another modification, the annular structure of the blood vessel holding unit 10 can be continuously arranged in the axial direction. As illustrated in Fig. 11, when the blood vessel holding unit 10 is expanded in the axial direction, the implantable medical device 1 can expand a region for applying mechanical stimulation to the blood vessel Bv along the traveling direction of the blood vessel Bv. In addition, by expanding the blood vessel holding unit 10 in the axial direction, it is also possible to apply mechanical stimulation to the blood vessel wall while effectively expanding the stenosis site of the blood vessel Bv as in a general stent.

As described above, the implantable medical device 1 of the first embodiment includes the blood vessel holding unit 10 that holds the blood vessel wall of the blood vessel Bv so as to press the blood vessel wall from inside, the coupling unit 20 that is connected to the first curved portion 11 at the proximal end of the blood vessel holding unit 10 and disposed at a position where the proximal end is displaced from the center of the blood vessel Bv (preferably, near the blood vessel wall), and the collecting unit 30 that extends from the proximal end portion of the coupling unit 20 toward the proximal end side along the axial direction and is caught by the collecting device 200 that collects the implantable medical device 1 from inside the living body. Thus, the implantable medical device 1 can be indwelled in the living body and then taken out of the living body using the collecting device 200 at a predetermined timing.

Further, the blood vessel holding unit 10 is arranged so that the phases in the circumferential direction around the central axis C of the first curved portions 11 and the second curved portions 12 arranged at both ends of the first connecting portion 13 are shifted from each other. Therefore, when the implantable medical device 1 is indwelled in the blood vessel Bv, the number of stimulation points to the blood vessel Bv can be increased, and the blood vessel Bv can be extended in different directions to apply effective mechanical stimulation to the baroreceptor as a stimulation target site.

Next, second to fifth embodiments as modification examples of the implantable medical device 1 according to the present invention will be described. Note that, in the following description of each of the embodiments, differences from the above-described embodiments will be mainly described, and constituent elements having functions equivalent to those of other embodiments will be denoted by the same or related reference numerals and will not be described in detail. In addition, the configuration, the members, the method of use, and the like may be the same as those in each embodiment. Further, the embodiments described in the first to fifth embodiments can be implemented in any combination without departing from the gist of the present invention.

### [Second Embodiment]

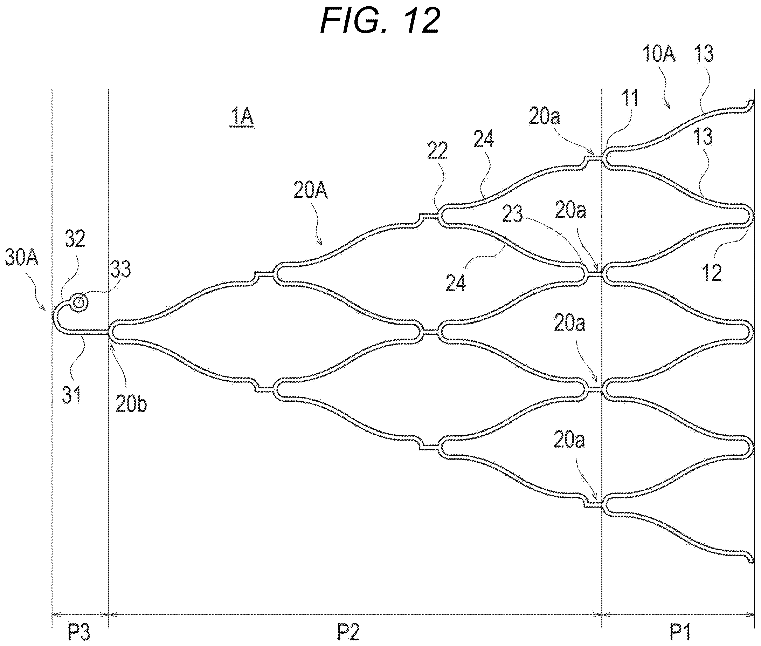

An implantable medical device 1A according to a second embodiment will be described with reference to Figs. 12 to 13B as appropriate.

The implantable medical device 1A of the second embodiment includes a blood vessel holding unit 10A, a coupling unit 20A, and a collecting unit 30A. As illustrated in Fig. 12, the blood vessel holding unit 10 of the implantable medical device 1A is a P1 portion, the coupling unit 20 is a P2 portion, and the collecting unit 30 is a P3 portion. The implantable medical device 1A is different from the implantable medical device 1 of the first embodiment in the shape of the coupling unit 20A.

As illustrated in Fig. 12, the blood vessel holding unit 10A has an annular structure including first curved portions 11, second curved portions 12, and first connecting portions 13 at least on the distal end side.

The coupling unit 20A includes at least one or more third curved portions 22 protruding toward the proximal end side, at least one or more fourth curved portions 23 protruding toward the distal end side, and at least one or more linear second connecting portions 24 connecting the third curved portions 22 and the fourth curved portions 23. The coupling unit 20A has a multistage structure in which the number of the third curved portions 22 and the fourth curved portions 23 gradually decreases from the distal end side toward the proximal end side. As illustrated in Fig. 11, the number of the third curved portions 22 and the fourth curved portions 23 of the coupling unit 20A gradually decreases to three, two, and one from the distal end side connected to the blood vessel holding unit 10A toward the proximal end side.

The fourth curved portion 23 located at the distal end of the coupling unit 20A is connected to the first curved portion 11 of the blood vessel holding unit 10A adjacent on the distal end side in the axial direction, and the fourth curved portion 23 located at the proximal end is connected to the third curved portion 22 of the coupling unit 20A adjacent on the distal end side in the axial direction. The third curved portion 22 located at the proximal end of the coupling unit 20A is disposed near the blood vessel wall of the blood vessel Bv. The third curved portion 22 located at the proximal end is connected to the collecting unit 30A.

The collecting unit 30A is provided to extend in the axial direction from the third curved portion 22 disposed near the blood vessel wall. The collecting unit 30A is disposed at a position (preferably, near the blood vessel wall) shifted from the center of the blood vessel Bv.

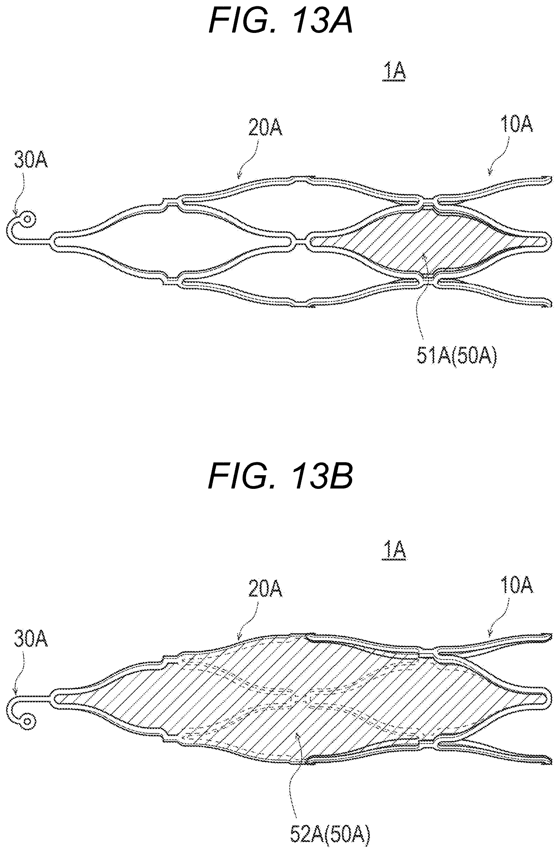

On the peripheral surface of the implantable medical device 1A, cells 50A (a first cell 51A and a second cell 52A), which are opening portions, are formed. As illustrated in Fig. 13A, in the implantable medical device 1A, a first cell 51A surrounded by the first connecting portions 13 of the blood vessel holding unit 10A and the coupling unit 20 is formed on the peripheral surface. As illustrated in Fig. 13B, in the implantable medical device 1A, a second cell 52A surrounded by the first connecting portions 13 of the blood vessel holding unit 10 and the coupling unit 20 is formed on the peripheral surface.

The first cell 51A and the second cell 52A are both formed on the peripheral surface of the implantable medical device 1, and have different cell areas. When the first cell 51A illustrated in Fig. 13A is compared with second cell 52A illustrated in Fig. 13B, the second cell 52A has a larger cell area than first cell 51A. As described above, since the implantable medical device 1A has the cells 50A having different cell areas on the peripheral surface, the passability of the blood flow can be improved.

As described above, the implantable medical device 1A of the second embodiment includes the coupling unit 20A having a multistage structure including the blood vessel holding unit 10 that holds the blood vessel wall of the blood vessel Bv so as to press the blood vessel wall from the inside, at least one or more third curved portions 22 that protrude toward the proximal end side, at least one or more fourth curved portions 23 that protrude toward the distal end side, and at least one or more linear second connecting portions 24 that connect the third curved portion 22 and the fourth curved portion 23, in which the number of the third curved portions 22 and the fourth curved portions 23 gradually decreases from the distal end side toward the proximal end side. The proximal end portion of the coupling unit 20A is disposed at a position shifted from the center of the blood vessel Bv (preferably, in the vicinity of the blood vessel wall), and is connected to the collecting unit 30 that extends toward the proximal end side along the axial direction and is caught by the collecting device 200 that collects the implantable medical device 1A from inside the living body. Thus, the implantable medical device 1A can be indwelled in the living body and then taken out of the living body using the collecting device 200 at a predetermined timing.

Further, the blood vessel holding unit 10A is arranged so that the phases in the circumferential direction around the central axis C of the first curved portion 11 and the second curved portion 12 arranged at both ends of the first connecting portion 13 are shifted from each other. Therefore, when the implantable medical device 1A is indwelled in the blood vessel Bv, the number of stimulation points to the blood vessel Bv can be increased, and the blood vessel Bv can be extended in different directions to apply effective mechanical stimulation to the baroreceptor as a stimulation target site.

### (Third Embodiment)

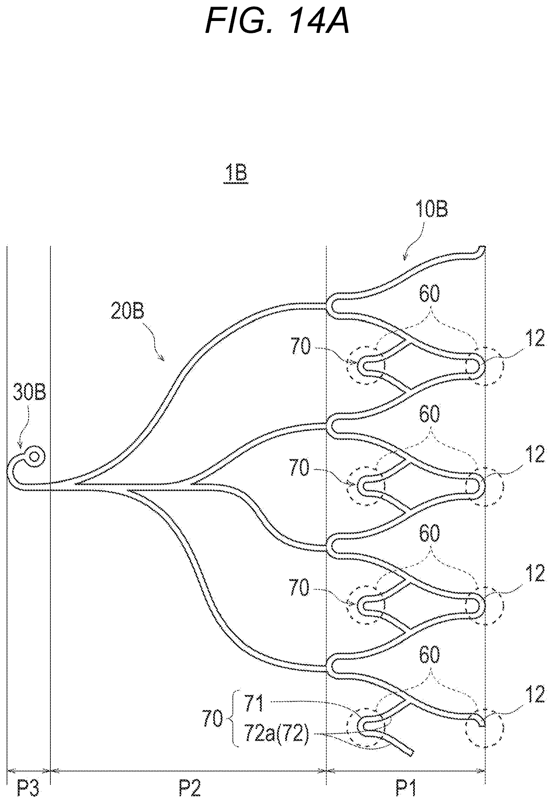

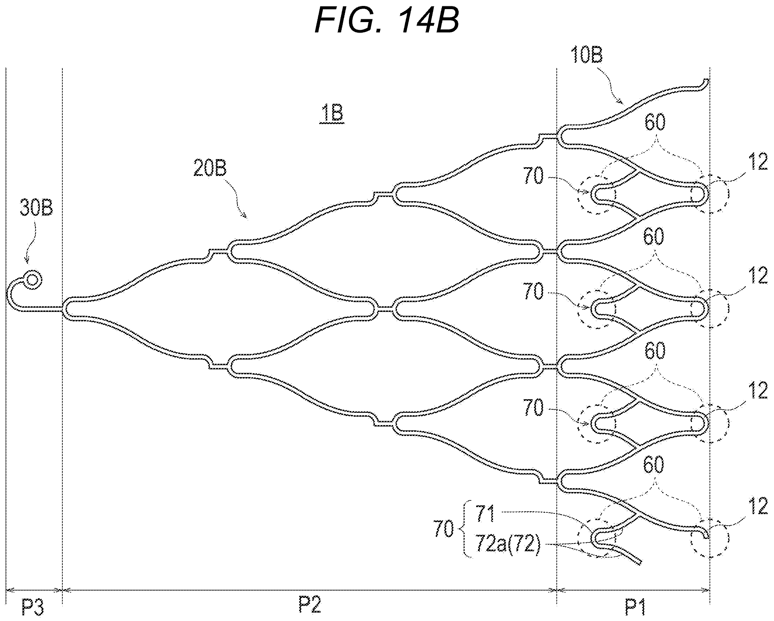

An implantable medical device 1B according to a third embodiment will be described with reference to Figs. 14A and 14B as appropriate.

As illustrated in Figs. 14A and 14B, the implantable medical device 1B of the third embodiment includes a blood vessel holding unit 10B, a coupling unit 20B, and a collecting unit 30B. As illustrated in Figs. 14A and 14B, the blood vessel holding unit 10 of the implantable medical device 1B is a P1 portion, the coupling unit 20 is a P2 portion, and the collecting unit 30 is a P3 portion.

As illustrated in Figs. 14A and 14B, the implantable medical device 1B is different from the other embodiments in that it includes protruding portions 60 (portions surrounded by a dotted line in the drawing) partially deformed and protruding toward the blood vessel wall side, and that it includes an accessory unit 70. Fig. 14A illustrates a form in which a plurality of accessory units 70 is provided in the implantable medical device 1 of the first embodiment, and Fig. 14B illustrates a form in which a plurality of accessory units 70 is provided in the implantable medical device 1A of the second embodiment.

The protruding portions 60 are formed by partially deforming the blood vessel holding unit 10B toward the blood vessel wall side. The protruding portions 60 can be configured by deforming the accessory units 70 protruding toward the proximal end side or the distal end side connected to the second curved portions 12 of the blood vessel holding unit 10B and the first connecting portions 13 of the blood vessel holding unit 10 toward the blood vessel wall side. At least one or more of the second curved portions 12 and the accessory units 70 that can function as the protruding portions 60 can be deformed to increase the contact point with the blood vessel wall.

The accessory units 70 have a V-shape including a base portion 71 and both leg portions 72 having a pair of leg portions 72a extending from a proximal end of the base portion 71, respective proximal ends of the leg portions 72a being individually connected to the first connecting portions 13 adjacent in the circumferential direction. The number and arrangement positions of the accessory units 70 are not particularly limited, and can be appropriately set so as to effectively stimulate the blood vessel Bv.

As described above, in the implantable medical device 1B of the third embodiment, since the blood vessel holding unit 10 includes the protruding portions 60 partially deformed and protruding toward the blood vessel wall side, the contact points with the blood vessel Bv can be increased as compared with the configuration not including the protruding portions 60, and the blood vessel Bv can be extended in the different directions to more effectively apply mechanical stimulation to the blood vessel Bv. Further, the implantable medical device 1 can further increase the stimulation points for the blood vessel Bv by providing the accessory units 70.

### [Fourth Embodiment]

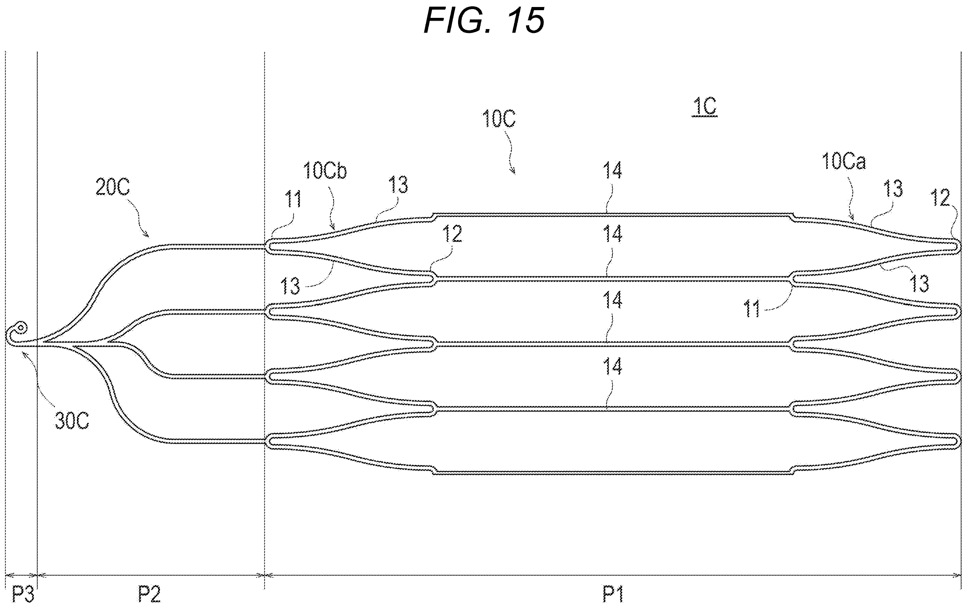

An implantable medical device 1C according to a fourth embodiment will be described with reference to Figs. 15 to 18 as appropriate.

As illustrated in Fig. 15, the implantable medical device 1C of the fourth embodiment includes a blood vessel holding unit 10C, a coupling unit 20C, and a collecting unit 30C. As illustrated in Figs. 15 to 18, the blood vessel holding unit 10 of the implantable medical device 1C is a P1 portion, the coupling unit 20 is a P2 portion, and the collecting unit 30 is a P3 portion. The implantable medical device 1C is different from the other embodiments in the shape of the blood vessel holding unit 10C.

The blood vessel holding unit 10C includes a distal end holding portion 10Ca having an annular structure and positioned at a distal end of the blood vessel holding unit 10C, a proximal end holding portion 10Cb having an annular structure and positioned at a proximal end of the blood vessel holding unit 10C, and a linear link portion 14 connecting the distal end holding portion 10Ca and the proximal end holding portion 10Cb. Further, in the blood vessel holding unit 10C, the number of the first curved portions 11 and the second curved portions 12 of the distal end holding portion 10Ca in the circumferential direction and the number of the first curved portions 11 and the second curved portions 12 of the proximal end holding portion 10Cb in the circumferential direction are the same.

The link portion 14 is at least one or more linear members connected between the distal end holding portion 10Ca and the proximal end holding portion 10Cb, and more specifically is at least one or more linear members connected between the first curved portion 11 of the distal end holding portion 10Ca and the second curved portion 12 of the proximal end holding portion 10Cb. Since the link portion 14 is connected between the first curved portion 11 of the distal end holding portion 10Ca and the second curved portion 12 of the proximal end holding portion 10Cb, a figure defined by connecting the link portion 14 and a figure defined by connecting the second curved portions 12 of the distal end holding portion 10Ca or a figure defined by connecting the first curved portions 11 of the proximal end holding portion 10Cb are quadrangles having substantially the same area, and have phases shifted from each other in the circumferential direction, and thus form an octagon when apex positions of the quadrangles are connected to each other as viewed from the axial distal end side (front side). Therefore, when the implantable medical device 1C is expanded, the contact points with the blood vessel wall increase, and mechanical stimulation can be applied to the blood vessel Bv in contact with the blood vessel walls. Further, stimulation points to the blood vessel Bv are increased linearly by the link portion 14, and the mechanical stimulation can be efficiently applied.

The link portion 14 can have forms illustrated in Figs. 15 to 18.

As illustrated in Fig. 15, the link portion 14 can be configured by a plurality of linear members extending in the axial direction. As illustrated in Fig. 15, the link portion 14 includes four linear members, and the four first curved portions 11 of the distal end holding portion 10Ca and the four second curved portions 12 of the proximal end holding portion 10Cb adjacent in the axial direction are individually connected to the link portion 14.

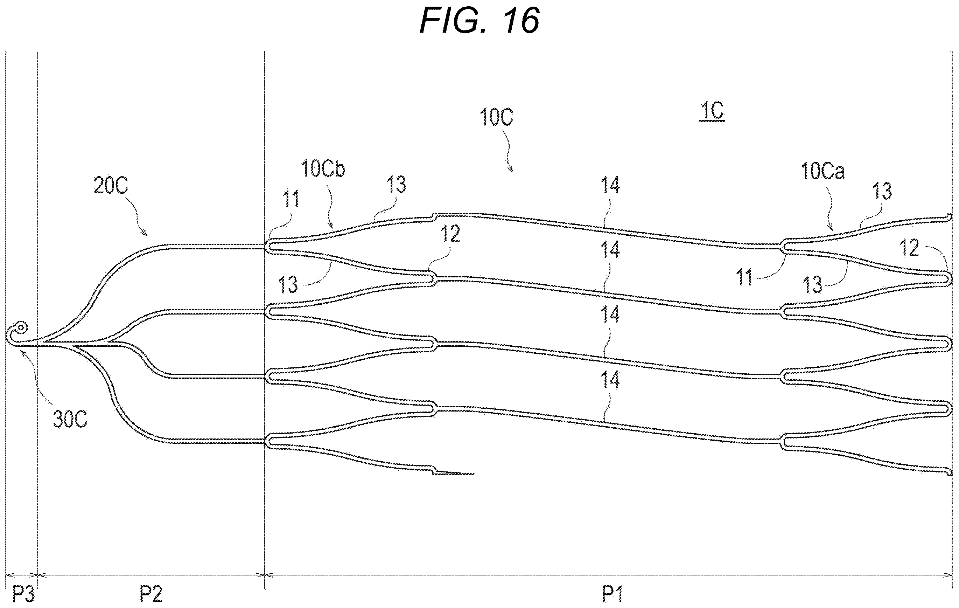

As illustrated in Fig. 16, the link portion 14 can be formed to extend in a twisted manner in a direction different from the central axis C. As illustrated in Fig. 16, the link portion 14 includes four linear members, and the four first curved portions 11 of the distal end holding portion 10Ca and the four second curved portions 12 of the proximal end holding portion 10Cb having phases shifted in the circumferential direction are individually connected to the link portion 14. Thus, since the stimulation points by the link portion 14 are at a twisted position in the axial direction of the blood vessel Bv, effective mechanical stimulation can be applied to the blood vessel Bv.

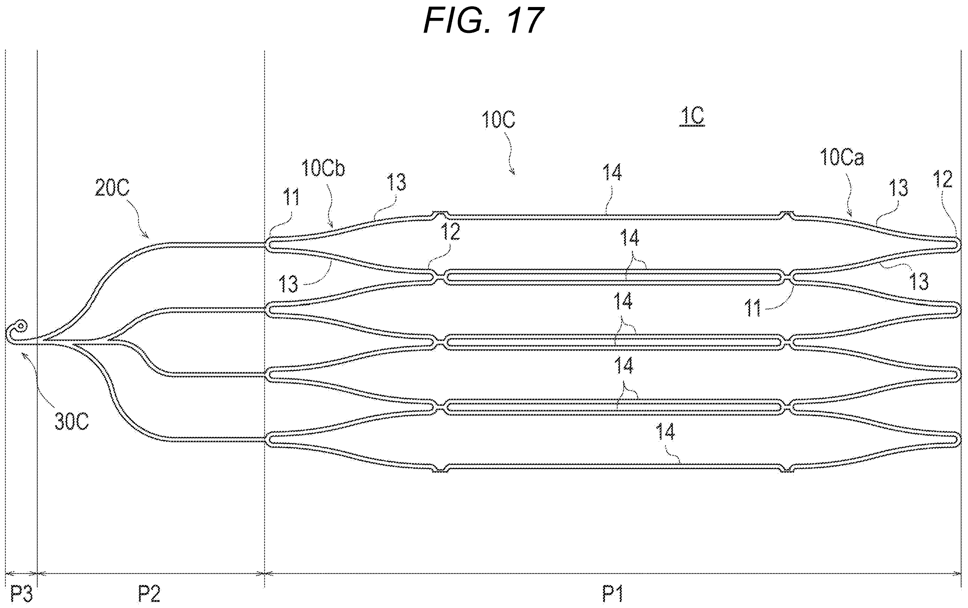

As illustrated in Fig. 17, the link portion 14 can have a configuration in which the first curved portions 11 of the distal end holding portion 10Ca and the second curved portions 12 of the proximal end holding portion 10Cb to be connected are connected by the plurality of linear members extending in the axial direction. As illustrated in Fig. 17, the link portion 14 includes two linear members as one set, and individually connects the four first curved portions 11 of the distal end holding portion 10Ca and the four second curved portions 12 of the proximal end holding portion 10Cb adjacent in the axial direction. Thus, the number of stimulation points by the link portion 14 is increased, and effective mechanical stimulation can be applied to the blood vessel Bv.

Note that the number of linear members of one set of the link portions 14 is not limited to two, and may be three or more. Further, the link portion 14 illustrated in Fig. 17 has a shape in which each linear member extends parallel to the axial direction after branching at the connection portion with the first curved portion 11 or the second curved portion 12, but each linear member may be directly connected individually between the first curved portion 11 and the second curved portion 12 to be connected.

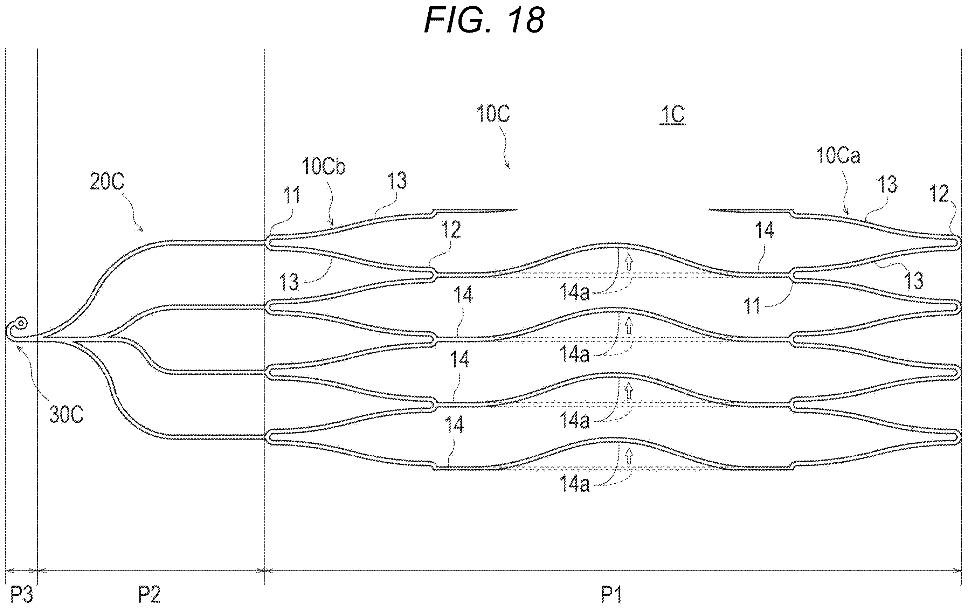

In addition, as illustrated in Fig. 18, the implantable medical device 1C can also be provided with a deformable portion 14a that deforms so as to partially protrude toward the blood vessel wall at the time of expansion on the link portion 14. The deformable portion 14a is not deformed by contact with the lumen of the catheter 110 of the delivery device 100 in a state (contracted state) before indwelling of the implantable medical device 1C. At this time, the link portion 14 maintains a substantially linear shape like a dotted line portion illustrated in Fig. 18. In a state (expanded state) after the implantation of the implantable medical device 1C, the deformable portion 14a is deformed so as to protrude toward the blood vessel wall side following the expansion of the implantable medical device 1C as illustrated in Fig. 18 (solid line portion in the drawing). Thus, when the link portion 14 comes into contact with the blood vessel wall, stimulativeness to the blood vessel Bv is further enhanced, and the mechanical stimulation can be effectively applied.

As described above, the implantable medical device 1C of the fourth embodiment includes the link portion 14 extending in the axial direction between the distal end holding portion 10Ca having an annular structure and the proximal end holding portion 10Cb also having an annular structure. The link portion 14 can include at least one or more linear members extending in the axial direction as illustrated in Fig. 15, a linear member extending in a twisted manner in a direction different from the central axis C as illustrated in Fig. 16, and a plurality of linear members connecting the first curved portions 11 of the distal end holding portion 10Ca and the second curved portions 12 of the proximal end holding portion 10Cb to be connected as illustrated in Fig. 17. Thus, in the implantable medical device 1C, mechanical stimulation can be applied to different positions of the blood vessel Bv by the first curved portions 11 and the second curved portions 12 of the distal end holding portion 10Ca and the first curved portions 11 and the second curved portions 12 of the proximal end holding portion 10Cb, and the mechanical stimulation can also be applied to the blood vessel Bv by the link portion 14.

In addition, in the implantable medical device 1C, as illustrated in Fig. 18, the link portion 14 is provided with the deformable portion 14a that deforms so as to partially protrude toward the blood vessel wall at the time of expansion, so that the stimulativeness to the blood vessel Bv is further enhanced and the mechanical stimulation can be effectively applied to the blood vessel Bv.

### [Fifth Embodiment]

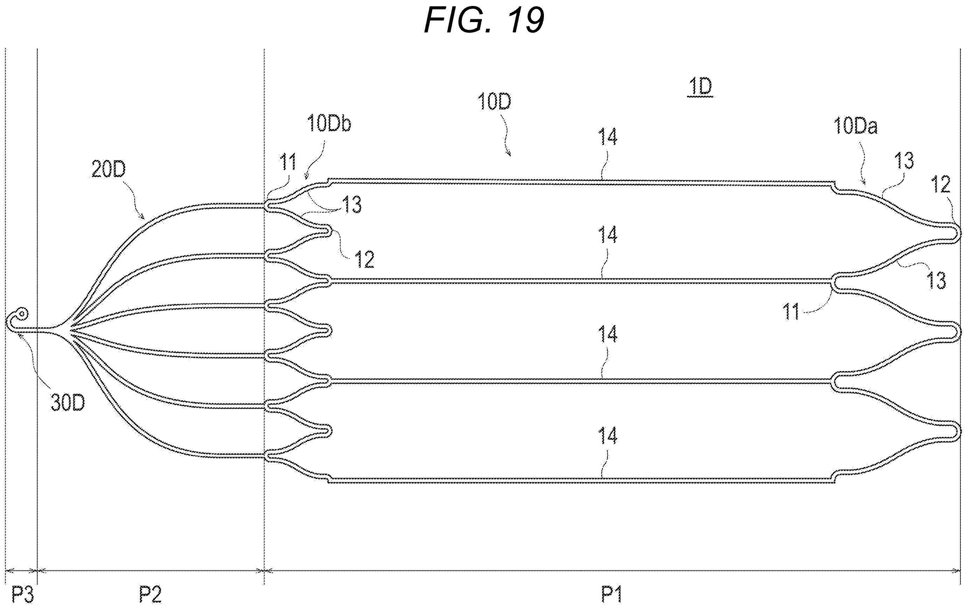

An implantable medical device 1D according to a fifth embodiment will be described with reference to Fig. 19.

As illustrated in Fig. 19, the implantable medical device 1D of the fifth embodiment includes a blood vessel holding unit 10D, a coupling unit 20D, and a collecting unit 30D. As illustrated in Fig. 19, the blood vessel holding unit 10 of the implantable medical device 1D is a P1 portion, the coupling unit 20 is a P2 portion, and the collecting unit 30 is a P3 portion.

As illustrated in Fig. 19, in the implantable medical device 1D, the blood vessel holding unit 10D includes a distal end holding portion 10Da having an annular structure and positioned at a distal end of the blood vessel holding unit 10D, a proximal end holding portion 10Db having an annular structure and positioned at a proximal end of the blood vessel holding unit 10D, and a linear link portion 14 connecting the distal end holding portion 10Da and the proximal end holding portion 10Db.

In the blood vessel holding unit 10D, the number of the first curved portions 11 and the second curved portions 12 of the distal end holding portion 10Da in the circumferential direction is different from the number of the first curved portions 11 and the second curved portions 12 of the proximal end holding portion 10Db in the circumferential direction. As illustrated in Fig. 19, in the implantable medical device 1D, the number of first curved portions 11 and the number of second curved portions 12 of the distal end holding portion 10Da are both three, and the number of first curved portions 11 and the number of second curved portions 12 of the proximal end holding portion 10Db are both six. Note that the number of the first curved portions 11 and the second curved portions 12 of the distal end holding portion 10Da in the circumferential direction and the number of the first curved portions 11 and the second curved portions 12 of the proximal end holding portion 10Db in the circumferential direction can be arbitrarily set.

As illustrated in Fig. 19, the link portion 14 includes a plurality of linear members, and one of the six first curved portions 11 of the distal end holding portion 10Da and one of the three second curved portions 12 of the proximal end holding portion 10Db adjacent in the axial direction are individually connected.

As described above, in the implantable medical device 1D of the fifth embodiment, the number of the first curved portions 11 and the second curved portions 12 of the distal end holding portion 10Da in the circumferential direction is different from the number of the first curved portions 11 and the second curved portions 12 of the proximal end holding portion 10Db in the circumferential direction. Thus, in the implantable medical device 1D, the number of contact points of the distal end holding portion 10Da with the blood vessel wall and the number of contact points of the proximal end holding portion 10Db with the blood vessel wall are also different. Therefore, in the implantable medical device 1D, the number and positions of the stimulation points by the distal end holding portion 10Da and the number and positions of the stimulation points by the proximal end holding portion 10Db can be arbitrarily set according to the shape of the blood vessel Bv or the like, and appropriate mechanical stimulation can be applied to the blood vessel Bv.

### [Operational Effects]

As described above, the implantable medical device 1 according to the present embodiment is indwelled in the blood vessel Bv, the implantable medical device 1 includes the blood vessel holding unit 10 capable of expanding and contracting to apply mechanical stimulation to the blood vessel wall of the blood vessel Bv, the blood vessel holding unit 10 has an annular structure in which a wavy linear component including at least one or more first curved portions 11 protruding toward the proximal end side, at least one or more second curved portions 11 protruding toward the distal end side, and at least one or more linear first connecting portions 13 connecting the first curved portion 11 and the second curved portion 12 is annular, the first curved portion 11 forms at least one or more first apex portions 11' in the radial direction of the central axis C of the annular structure, the second curved portion 12 forms at least one or more second apex portions 12' in the radial direction of the central axis C of the annular structure, and the first apex portion 11' and the second apex portion 12' are arranged to be shifted in the circumferential direction around the central axis C of the annular structure and in the long axis direction of the central axis C of the annular structure.

With such a configuration, in the implantable medical device 1, the first apex portion 11' formed by the first curved portion 11 constituting the blood vessel holding unit 10 and the second apex portion 12' formed by the second curved portion 12 are arranged so as to be shifted in the circumferential direction around the central axis C of the annular structure and the long axis direction of the central axis C of the annular structure, and thus, when the implantable medical device 1 is indwelled in the blood vessel Bv, it is possible to increase the number of stimulation points to the blood vessel Bv, and the blood vessel Bv can be extended in different directions to apply effective mechanical stimulation to the baroreceptor as a stimulation target site.

In the implantable medical devices 1C and 1D according to the present embodiment, the blood vessel holding unit 10 may be configured to include distal end holding portions 10Ca and 10Da having the annular structure and positioned at the distal ends of the blood vessel holding unit 10, proximal end holding portions 10Cb and 10Db having the annular structure and positioned at the proximal ends of the blood vessel holding unit 10, and at least one or more linear link portions 14 connecting the first curved portions 11 of the distal end holding portions 10Ca and 10Db and the second curved portions 12 of the proximal end holding portions 10Cb and 10Db. In addition, in the blood vessel holding unit 10C, the number of the first curved portions 11 and the second curved portions 12 of the distal end holding portion 10Ca in the circumferential direction and the number of the first curved portions 11 and the second curved portions 12 of the proximal end holding portion 10Cb in the circumferential direction may be the same. Further, in the blood vessel holding unit 10D, the number of the first curved portions 11 and the second curved portions 12 of the distal end holding portion 10Da in the circumferential direction may be different from the number of the first curved portions 11 and the second curved portions 12 of the proximal end holding portion 10Db in the circumferential direction.

With such a configuration, in the implantable medical devices 1C and 1D, mechanical stimulation can be applied to different positions of the blood vessel Bv by the first curved portions 11 and the second curved portions 12 of the distal end holding portions 10Ca and 10Da and the first curved portions 11 and the second curved portions 12 of the proximal end holding portions 10Cb and 10Db, and mechanical stimulation can also be applied to the blood vessel Bv by the link portion 14. In addition, if the number of the first curved portions 11 and the second curved portions 12 of the distal end holding portion 10Da in the circumferential direction is different from the number of the first curved portions 11 and the second curved portions 12 of the proximal end holding portion 10Db in the circumferential direction, the number and positions of the stimulation points by the distal end holding portion 10Da and the number and positions of the stimulation points by the proximal end holding portion 10Db can be arbitrarily set according to the shape of the blood vessel Bv or the like, and appropriate mechanical stimulation can be applied to the blood vessel **Bv.**

In the implantable medical device 1C according to the present embodiment, the link portion 14 may be configured to include a deformable portion 14a that deforms so as to partially protrude toward the blood vessel wall at the time of expansion.

With such a configuration, the stimulativeness to the blood vessel Bv by the link portion 14 is further enhanced, and effective mechanical stimulation can be applied to the blood vessel **Bv.**

In the implantable medical device 1C according to the present embodiment, the link portion 14 may be configured to connect the first curved portion 11 of the distal end holding portion 10Ca and the second curved portion 12 of the proximal end holding portion 10Cb to be connected by a plurality of linear members extending in the axial direction.

In the implantable medical device 1C according to the present embodiment, the link portion 14 may be configured to extend in a twisted manner in a direction different from the central axis C.

With such a configuration, the stimulation point by the link portion 14 is located at a twisted position in the axial direction of the blood vessel Bv, so that effective mechanical stimulation can be applied to the blood vessel Bv.

The implantable medical devices 1A and 1B according to the present embodiment may be configured to include at least one accessory unit 70 having a V-shape including a base portion 71 and both leg portions 72 having a pair of leg portions 72a extending from a proximal end of the base portion 71, respective proximal ends of the leg portions 72a being individually connected to the first connecting portions 11 adjacent in the circumferential direction.

With such a configuration, it is possible to further increase the stimulation points of the blood vessel holding unit 10 with respect to the blood vessel Bv.

In the implantable medical device 1B according to the present embodiment, the blood vessel holding unit 10B may be configured to include a protruding portion 60 partially deformed and protruding toward the blood vessel wall side.

With such a configuration, the contact points of the blood vessel holding unit 10B with the blood vessel Bv can be increased, and the blood vessel Bv can be extended in different directions to more effectively apply mechanical stimulation to the blood vessel Bv.

The implantable medical device 1 according to the present embodiment may have a configuration of at least 1 or more, preferably 2 or more, more preferably 2 or more and 20 or less, and still more preferably 4 or more and 8 or less.

With such a configuration, the implantable medical device 1 can appropriately hold the blood vessel Bv from the inside and effectively apply mechanical stimulation.

The implantable medical device 1 according to the present embodiment may be configured to include the collecting unit 30 to be caught by the collecting device 200 that collects the implantable device 1 from the living body on the proximal end side of the blood vessel holding unit 10.

With such a configuration, the implantable medical device 1 can be indwelled in the living body and then taken out of the living body using the collecting device 200 at a predetermined timing.

A delivery device 100 according to the present embodiment is a device that delivers an implantable medical device 1 to an indwelling position in a living body and indwells the implantable medical device 1 in the living body, in which the implantable medical device 1 includes a coupling unit 20 in which a distal end portion is connected to a proximal end of a blood vessel holding unit 10 adjacent in an axial direction, and a proximal end portion is aggregated so as to gradually reduce in diameter from the proximal end of the blood vessel holding unit 10 toward a proximal end side and arranged at a position shifted from a center of a blood vessel Bv, the delivery device 100 includes a catheter 110 having a lumen and a delivery member 120 having an attachment portion 122 that is relatively movable in the axial direction with respect to the catheter 110 and to which the implantable medical device 1 is attached, the attachment portion 120 includes a support portion 122a having an inclined surface 122e formed to match an outer shape of the coupling unit 20 of the implantable medical device 1 and supporting the coupling unit 20, a column portion 122b extending from the inclined surface 122e of the support portion 122a toward the distal end side in the axial direction and inserted into the implantable medical device **1,** and a pressing portion 122c provided at a distal end of the column portion 122b and in contact with the distal end side of the blood vessel holding unit 10 of the implantable medical device 1.

With such a configuration, when the implantable medical device 1 is attached to the attachment portion 122, it is attached in a state in which an outer surface of the coupling unit 20 and the inclined surface 122e of the support portion 122a are brought into contact with each other, the column portion 122b is inserted into the implantable medical device **1,** and the distal end of the blood vessel holding unit 10 and the pressing portion 122c are brought into contact with each other. Thus, since the implantable medical device 1 is disposed between the support portion 122a and the pressing portion 122c in a state of being inserted into the column portion 122b, both end sides are held, and falling off during delivery can be prevented. Further, since the implantable medical device 1 is attached to the attachment portion 122 in a state of being inserted into the column portion 122b, displacement in the radial direction at the time of delivery is suppressed, and the implantable medical device 1 can be smoothly delivered without being caught in a lumen of the catheter 110 during delivery.

Hereinafter, a stent according to another embodiment will be disclosed. A stent 1 according to the another embodiment can function as the implantable medical device 1.

As a conventional technique, Japanese Patent Application Laid-Open No. 2006-55330 discloses a stent including a cylindrical main body portion that is freely reduced and expanded in diameter in order to be capable of being indwelled in a biological lumen such as a blood vessel for a certain period of time and thereafter stored in a catheter and collected to the outside of the body without damaging an inner wall of the biological lumen, a tail portion that is provided following the main body portion and is enlarged in diameter to be tapered in a normal state in which the diameter is freely reduced and expanded, a filament bundle provided following the tail portion, and an engaging portion formed by flexing or bending of the filament bundle provided following a rear end of the filament bundle, in which the stent is formed into a braided string structure by spirally twisting one or a plurality of filaments.

In the stent of the above literature, since the engaging portion extending from the tapered tail portion is located on the central axis of the blood vessel in a state of being indwelled in the blood vessel, there is a possibility that blood flow is inhibited and the risk of occurrence of blood flow disturbance increases. Further, in the stent of Patent Literature **1,** since the engaging portion is disposed at the center of the blood vessel, thrombus is likely to occur. For example, when the stent is indwelled in the vicinity of a bifurcation of a blood vessel where thrombus formation is likely to be promoted, the risk of occurrence of thrombus may be increased.

The stent 1 of at least one embodiment of the present invention has been made in view of the above circumstances, and specifically, an object thereof is to have a configuration that can be collected after being indwelled in a biological lumen such as a blood vessel, and to reduce the risk of inhibiting blood flow or generating thrombus at the time of indwelling.

The stent 1 according to the present invention is a medical instrument that is indwelled in a biological lumen such as a blood vessel Bv, expands a stenosis site or a blockage site to secure a lumen of the blood vessel Bv in order to treat various diseases caused by stenosis or blockage, or applies mechanical stimulation to a stimulation target site (for example, a baroreceptor of the blood vessel Bv) of the biological lumen such as the blood vessel Bv, and expands and presses the stimulation target site from the inside of a lumen wall (blood vessel wall) to stimulate the stimulation target site in order to treat a predetermined disease by acting on the vagus nerve.

An expansion portion of the stent 1 in another embodiment is the blood vessel holding unit 10, and has an annular structure including linear components as illustrated in Figs. 1 and 2. The blood vessel holding unit 10 has an annular structure at least on the proximal end side.

The stent 1 according to the another embodiment of the present invention is achieved by any of the following configurations (1) to (15).
(1) A stent arranged in a biological lumen, the stent including an annular expansion portion including a linear component, a coupling unit in which a distal end portion is connected to a proximal end of the expansion portion adjacent in an axial direction, and a proximal end portion is aggregated so as to gradually reduce in diameter from the proximal end of the expansion portion toward a proximal end side and arranged at a position shifted from a center of the biological lumen, and a collecting unit that extends from the proximal end portion of the coupling unit toward the proximal end side along the axial direction and caught by a collecting device that collects the stent from inside of the living body.
(2) The stent according to (1) above, in which the expansion portion has an annular structure in which a wavy linear component including at least one or more first curved portions protruding toward the proximal end side, at least one or more second curved portions protruding toward a distal end side, and at least one or more linear first connecting portions connecting the first and second curved portions, and the coupling unit is connected to the first curved portion of the expansion portion.
(3) The stent according to (2) above, in which the coupling unit includes a plurality of linear members elongated in the axial direction, distal end portions of the plurality of linear members are individually connected to the plurality of first curved portions of the expansion portion, and proximal end portions of the plurality of linear members are aggregated near a lumen wall of the biological lumen.
(4) The stent according to (2) above, in which the expansion portion has the annular structure at least on a distal end side, the coupling unit including at least one or more third curved portions protruding toward a proximal end side, at least one or more fourth curved portions protruding toward the distal end side, and at least one or more linear second connecting portions connecting the third curved portion and the fourth curved portion, the coupling unit has a multistage structure in which the number of the third curved portions and the fourth curved portions decreases stepwise from the distal end side toward the proximal end side, the fourth curved portion located at a distal end is connected to the first curved portion of the expansion portion adjacent on the distal end side in the axial direction, the fourth curved portion located at a proximal end is connected to the third curved portion of the coupling unit adjacent on the distal end side in the axial direction, and the third curved portion located at a proximal end of the coupling unit is arranged near a lumen wall of the biological lumen.
(5) The stent according to any one of (2) to (4) above, in which the stent has a first cell surrounded by the first connecting portion and the coupling unit and a second cell having a cell area different from that of the first cell on a peripheral surface.
(6) The stent according to any one of (1) to (4) above, in which the collecting unit includes an extending portion connected to the proximal end portion of the coupling unit and extending in the axial direction, and an engaging portion bent in a U shape from a proximal end of the extending portion toward a distal end side.
(7) The stent according to (6) above, in which the engaging portion is bent in a direction away from the lumen wall of the biological lumen.
(8) The stent according to (6) above, in which the engaging portion is arranged along a lumen wall surface of the biological lumen.
(9) The stent according to any one of (1) to (4) above, in which the collecting unit is configured to be magnetically attractable to the collecting device.
(10) The stent according to any one of (1) to (4) above, in which the collecting unit and/or the expansion portion includes a marker portion having X-ray contrast property.
(11) The stent according to any one of (2) to (4) above, in which numbers of the first curved portions and the second curved portions formed are at least two or more.
(12) The stent according to any one of (2) to (4) above, in which numbers of both the first curved portions and the second curved portions formed are two or more and twenty or less.
(13) The stent according to any one of (2) to (4) above, in which numbers of both the first curved portions and the second curved portions formed are four or more and eight or less.
(14) The stent according to any one of (1) to (4) above, in which the stent is disposed in a carotid sinus of an internal carotid artery, and applies mechanical stimulation to a baroreceptor in the carotid sinus by the expansion portion.
(15) A delivery device that delivers the stent according to (1) to an indwelling position in a living body, in which the delivery device includes the delivery device includes a catheter having a lumen and a delivery member having an attachment portion that is relatively movable in an axial direction with respect to the catheter and to which the stent is attached, and the attachment portion includes a support portion having an inclined surface formed to match an outer shape of the coupling unit of the stent and supporting the coupling unit, a column portion extending from the inclined surface of the support portion toward a distal end side in the axial direction and inserted into the stent, and a pressing portion provided at a distal end of the column portion and in contact with a distal end side of the expansion portion of the stent.

The inventions (1) to (15) have the following effects.

With the stent 1 of (1) above, after being indwelled in the living body, the stent 1 can be engaged with and caught by the collecting unit 30 using the collecting device 200, and thus can be taken out of the living body at a predetermined timing. Further, since the proximal end portion of the coupling unit 20 to which the collecting unit 30 is connected is located at a position shifted from the center of the blood vessel Bv, the risk of inhibiting the blood flow and generating thrombus is reduced.

With the stent 1 of (2) above, since all of the first curved portions 11 located at the proximal ends of the blood vessel holding units 10 as the expansion portion are connected to the coupling unit 20, there is no site protruding on the proximal end side (P2 portion and P3 portion) of the stent 1. That is, the proximal end side of the stent 1 is configured so that the stent 1 does not exist in a part of the circumferential direction of the central axis C, and the distal end side of the stent 1 is configured so that the stent 1 exists in the entire circumferential direction of the central axis C, and the stent 1 has a shape of an obliquely cut circular cylinder (a circular cylinder with one end obliquely cut off). Therefore, when the stent 1 is indwelled in the blood vessel Bv, the stent 1 does not cover other blood vessels Bv in the vicinity. Therefore, when the stent 1 is indwelled in the carotid sinus of the internal carotid artery, the stent 1 can be indwelled so that a part of the blood vessel holding unit 10 does not cover the external carotid artery branching from the common carotid artery.

With the stent 1 of (3) and (4) above, since all of the first curved portions 11 located at the proximal ends of the blood vessel holding units 10 and 10A are connected to the coupling unit 20 and the coupling unit 20A, there is no site protruding to the proximal end side (P2 portion and P3 portion) of the stent 1. That is, the proximal end side of the stent 1 is configured so that the stent 1 does not exist in a part of the circumferential direction of the central axis C, and the distal end side of the stent 1 is configured so that the stent 1 exists in the entire circumferential direction of the central axis C, and the stent 1 has a shape of an obliquely cut circular cylinder (a circular cylinder with one end obliquely cut off). Therefore, when the stents 1 and 1A are indwelled in the blood vessel Bv, the stents 1 and 1A can be indwelled without covering other nearby blood vessels Bv. Further, since the third curved portion 22 located at the proximal ends of the coupling units 20 and 20A is gathered or disposed near the blood vessel wall, the collecting units 30 and 30A connected to the coupling units 20 and 20A can also be disposed near the blood vessel wall. Therefore, when the stents 1 and 1A are indwelled in the blood vessel Bv, the risk of blood flow inhibition and thrombus generation by the collecting units 30 and 30A is reduced.

With the stent 1 of (5) above, since the cell areas of the first cells 51 and 51A and the second cells 52 and 52A formed on the peripheral surfaces of the stents 1 and 1A are different from each other, favorable blood flow passability can be obtained as compared with a form in which the cell area is uniform.

With the stent 1 of (6) above, since the collecting unit 30 has a hook shape having the extending portion 31 and the engaging portion 32 connected to the proximal end side of the coupling unit 20, it is possible to easily engage with the catching portion 220 of the collecting device 200.

With the stent 1 of (7) above, the engaging portion 32 is bent to be away from the blood vessel wall of the blood vessel Bv, so that the collection operation by the collecting device 200 can be easily performed.

With the stent 1 of (8) above, when the engaging portion 32 is disposed along the blood vessel wall of the blood vessel Bv, the engaging portion can be disposed without protruding in the radial direction of the blood vessel Bv, so that the risk of occurrence of thrombus is reduced.

With the stent 1 of (9) above, since it is magnetically attractable in addition to the engagement with the engaging portion 32 and the catching portion 220, the stent 1 is prevented from falling off during the collecting operation, and the stent 1 can be reliably recovered.

With the stent 1 of (10) above, since the position can be confirmed using a modality or the like when the stent 1 is indwelled in the blood vessel Bv, the stent 1 can be indwelled at a desired position with high accuracy.

With the stent 1 of (11), (12), and (13) above, the stent 1 can appropriately hold the blood vessel Bv from the inside and effectively apply mechanical stimulation.

With the stent 1 of (15) above, when the stent 1 is attached to the attachment portion 122, the stent 1 is attached in a state in which the outer surface of the coupling unit 20 and the inclined surface 122e of the support portion 122a are brought into contact with each other, the column portion 122b is inserted into the stent **1,** and the distal end of the blood vessel holding unit 10 and the pressing portion 122c are brought into contact with each other. Thus, since the stent 1 is arranged between the support portion 122a and the pressing portion 122c in a state of being inserted into the column portion 122b, both end sides are held, and falling off during delivery can be prevented. Further, since the stent 1 is attached to the attachment portion 122 in a state of being inserted into the column portion 122b, displacement in the radial direction at the time of delivery is suppressed, and the stent 1 can be smoothly delivered without being caught by the lumen of the catheter 110 during delivery.

With regard to the invention of the another embodiment described above, the following supplementary notes are further disclosed.

(Supplementary note 1) A stent arranged in a biological lumen, the stent including an annular expansion portion including a linear component, a coupling unit in which a distal end portion is connected to a proximal end of the expansion portion adjacent in an axial direction, and a proximal end portion is aggregated so as to gradually reduce in diameter from the proximal end of the expansion portion toward a proximal end side and arranged at a position shifted from a center of the biological lumen, and a collecting unit that extends from the proximal end portion of the coupling unit toward the proximal end side along the axial direction and caught by a collecting device that collects the stent from inside of the living body.

(Supplementary note 2) The stent according to (Supplementary note 1) above, in which the expansion portion has an annular structure in which a wavy linear component including at least one or more first curved portions protruding toward the proximal end side, at least one or more second curved portions protruding toward a distal end side, and at least one or more linear first connecting portions connecting the first and second curved portions, and the coupling unit is connected to the first curved portion of the expansion portion.

(Supplementary note 3) The stent according to (Supplementary note 1) or (Supplementary note 2) above, in which the coupling unit includes a plurality of linear members elongated in the axial direction, distal end portions of the plurality of linear members are individually connected to the plurality of first curved portions of the expansion portion, and proximal end portions of the plurality of linear members are aggregated near a lumen wall of the biological lumen.

(Supplementary note 4) The stent according to (Supplementary note 1) or (Supplementary note 2) above, in which the expansion portion has the annular structure at least on a distal end side, the coupling unit including at least one or more third curved portions protruding toward a proximal end side, at least one or more fourth curved portions protruding toward the distal end side, and at least one or more linear second connecting portions connecting the third curved portion and the fourth curved portion, the coupling unit has a multistage structure in which the number of the third curved portions and the fourth curved portions decreases stepwise from the distal end side toward the proximal end side, the fourth curved portion located at a distal end is connected to the first curved portion of the expansion portion adjacent on the distal end side in the axial direction, the fourth curved portion located at a proximal end is connected to the third curved portion of the coupling unit adjacent on the distal end side in the axial direction, and the third curved portion located at a proximal end of the coupling unit is arranged near a lumen wall of the biological lumen.

(Supplementary note 5) The stent according to any one of (Supplementary note 2) to (Supplementary note 4) above, in which the stent has a first cell surrounded by the first connecting portion and the coupling unit and a second cell having a cell area different from that of the first cell on a peripheral surface.

(Supplementary note 6) The stent according to any one of (Supplementary note 1) to (Supplementary note 5) above, in which the collecting unit includes an extending portion connected to the proximal end portion of the coupling unit and extending in the axial direction, and an engaging portion bent in a U shape from a proximal end of the extending portion toward a distal end side.

(Supplementary note 7) The stent according to (Supplementary note 6) above, in which the engaging portion is bent in a direction away from the lumen wall of the biological lumen.

(Supplementary note 8) The stent according to (Supplementary note 6) above, in which the engaging portion is arranged along a lumen wall surface of the biological lumen.

(Supplementary note 9) The stent according to any one of (Supplementary note 1) to (Supplementary note 8) above, in which the collecting unit is configured to be magnetically attractable to the collecting device.

(Supplementary note 10) The stent according to any one of (Supplementary note 1) to (Supplementary note 9) above, in which the collecting unit and/or the expansion portion includes a marker portion having X-ray contrast property.

(Supplementary note 11) The stent according to (Supplementary note 2) or (Supplementary note 5) above, in which numbers of the first curved portions and the second curved portions formed are at least two or more.

(Supplementary note 12) The stent according to any one of (Supplementary note 2) or (Supplementary note 5) above, in which numbers of both the first curved portions and the second curved portions formed are two or more and twenty or less.

(Supplementary note 13) The stent according to any one of (Supplementary note 2) or (Supplementary note 5) above, in which numbers of both the first curved portions and the second curved portions formed are four or more and eight or less.

(Supplementary note 14) The stent according to any of (Supplementary note 1) to (Supplementary note 13) above, the stent is disposed in a carotid sinus of an internal carotid artery, and applies mechanical stimulation to a baroreceptor in the carotid sinus by the expansion portion.

(Supplementary note 15) A delivery device that delivers the stent according to any one of (Supplementary note 1) to (Supplementary note 14) to an indwelling position in a living body, in which the delivery device includes the delivery device includes a catheter having a lumen and a delivery member having an attachment portion that is relatively movable in an axial direction with respect to the catheter and to which the stent is attached, and the attachment portion includes a support portion having an inclined surface formed to match an outer shape of the coupling unit of the stent and supporting the coupling unit, a column portion extending from the inclined surface of the support portion toward a distal end side in the axial direction and inserted into the stent, and a pressing portion provided at a distal end of the column portion and in contact with a distal end side of the expansion portion of the stent.

The present application is based on Japanese Patent Application No. 2022-194642 filed on December 6, 2022 and Japanese Patent Application No. 2022-194643 filed on December 6, 2022, the disclosures of which are incorporated herein by reference in their entireties.

### Reference Signs List

1, 1A to 1D Implantable medical device
10, 10A to 10D Blood vessel holding unit
10Ca, 10Da Distal end holding portion
10Cb, 10Db Proximal end holding portion
11 First curved portion
11' First apex portion
12 Second curved portion
12' Second apex portion
13 Second connecting portion
14 Link portion (14a deformable portion)
20, 20A to 20D Coupling unit
21 Linear member
22 Third curved portion
23 Fourth curved portion
24 Second connecting portion
30, 30A to 30D Collecting unit
31 Extending portion
32 Engaging portion
33 Magnetic portion
34 Corner
35 Pressing unit
36 Finger hooking portion
40, 113, 122d Marker portion
50, 50A Cell
51, 51A First cell
52, 52B Second cell
60 Protruding portion
61 First cover portion
62 Second cover portion
70 Accessory unit
71 Base portion
72 Both leg portions (72a leg portion)
73 Third cover storage portion
74 Fourth cover storage portion
100 Delivery device
110 Catheter
120 Delivery member
122 Attachment portion (122a support portion, 122b column portion, 122c pressing portion, 122e inclined surface)
200 Collecting device
Bv Blood vessel
C Central axis

## Claims

1. An implantable medical device indwelled in a blood vessel, wherein
the implantable medical device comprises a blood vessel holding unit that is capable of expanding and contracting to apply mechanical stimulation to a blood vessel wall of the blood vessel,
the blood vessel holding unit has an annular structure in which a wavy linear component including at least one or more first curved portions protruding toward a proximal end side, at least one or more second curved portions protruding toward a distal end side, and at least one or more linear first connecting portions connecting the first curved portion and the second curved portion is annular, and
the first curved portion forms at least one or more first apex portions in a radial direction of a central axis of the annular structure, the second curved portion forms at least one or more second apex portions in the radial direction of the central axis of the annular structure, and the first apex portion and the second apex portion are arranged to be shifted in a circumferential direction around the central axis of the annular structure and in a long axis direction of the central axis of the annular structure.

2. The implantable medical device according to claim **1,** wherein
the blood vessel holding unit includes:
a distal end holding portion having the annular structure and positioned at a distal end of the blood vessel holding unit;
a proximal end holding portion having the annular structure and positioned at a proximal end of the blood vessel holding unit; and
at least one or more linear link portions connecting the first curved portion of the distal end holding portion and the second curved portion of the proximal end holding portion.

3. The implantable medical device according to claim 2, wherein in the blood vessel holding unit, a number of the first curved portions and the second curved portions of the distal end holding portion in the circumferential direction and a number of the first curved portions and the second curved portions of the proximal end holding portion in the circumferential direction are same.

4. The implantable medical device according to claim 2, wherein in the blood vessel holding unit, a number of the first curved portions and the second curved portions of the distal end holding portion in the circumferential direction and a number of the first curved portions and the second curved portions of the proximal end holding portion in the circumferential direction are different.

5. The implantable medical device according to any one of claims 2 to 4, wherein the link portion includes a deformable portion that deforms so as to partially protrude toward the blood vessel wall at a time of expansion.

6. The implantable medical device according to any one of claims 2 to 4, wherein the link portion connects the first curved portion of the distal end holding portion and the second curved portion of the proximal end holding portion to be connected by a plurality of linear members extending in the axial direction.

7. The implantable medical device according to any one of claims 2 to 4, wherein the link portion extends in a twisted manner in a direction different from the central axis.

8. The implantable medical device according to claim 1, comprising at least one accessory unit having a V-shape including a base portion and both leg portions having a pair of leg portions extending from a proximal end of the base portion, respective proximal ends of the leg portions being individually connected to the first connecting portions adjacent in the circumferential direction.

9. The implantable medical device according to claim 1, wherein the blood vessel holding unit includes a protruding portion partially deformed and protruding toward the blood vessel wall side.

10. The implantable medical device according to any one of claims 1 to 4, wherein numbers of the first curved portions and the second curved portions formed are at least two or more.

11. The implantable medical device according to any one of claims 1 to 4, wherein numbers of both the first curved portions and the second curved portions formed are two or more and twenty or less.

12. The implantable medical device according to any one of claims 1 to 4, wherein numbers of both the first curved portions and the second curved portions formed are four or more and eight or less.

13. The implantable medical device according to claim 1, wherein the blood vessel is a carotid sinus of an internal carotid artery, and the blood vessel holding unit applies mechanical stimulation to a baroreceptor at the carotid sinus.

14. The implantable medical device according to claim 1, comprising a collecting unit to be caught by a collecting device that collects the implantable device from inside a living body on a proximal end side of the blood vessel holding unit.

15. A delivery device that delivers the implantable medical device according to claim 1 to an indwelling position in a living body and indwells the implantable medical device in the living body, wherein
the implantable medical device includes a coupling unit in which a distal end portion is connected to a proximal end of the blood vessel holding unit adjacent in an axial direction, and a proximal end portion is aggregated so as to gradually reduce in diameter from the proximal end of the blood vessel holding unit toward a proximal end side and arranged at a position shifted from a center of the blood vessel,
the delivery device includes a catheter having a lumen and a delivery member having an attachment that is relatively movable in the axial direction with respect to the catheter and to which the implantable medical device is attached, and
the attachment portion includes a support portion having an inclined surface formed to match an outer shape of the coupling unit of the implantable medical device and supporting the coupling unit, a column portion extending from the inclined surface of the support portion toward a distal end side in the axial direction and inserted into the implantable medical device, and a pressing portion provided at a distal end of the support portion and in contact with a distal end side of the blood vessel holding unit of the implantable medical device.
